# EUROPEAN PATENT APPLICATION

(11) **EP 4 606 391 A1**
(43) Date of publication of application: **27.08.2025**
(21) Application number: 25158869.5
(22) Date of filing: 19.02.2025
(51) Int. Cl.: A61L 2/00, G08B 21/24, G16H 40/20

(54) **FLUID DISPENSER WITH AUTOMATIC FLUID COMPOSITION ADJUSTMENT**

(30) Priority: 23.02.2024 US 202463557293 P
(71) Applicant: OP-Hygiene IP GmbH, 4704 Niederbipp (CH)
(72) Inventor: Ophardt, Heiner, 4422 Arisdorf (CH); Steltenkamp, Siegfried, 53229 Bonn (DE); Claudinon, Julie, 79108 Freiburg (DE); Jones, Andrew, St. Anns, L0R 1Y0 (CA); Lang, Albrecht, 4704 Niederbipp (CH)
(74) Representative: Grünecker Patent- und Rechtsanwälte PartG mbB

(57) **Abstract**

A hand cleaning system comprising a hand cleaning fluid dispenser configured to dispense an output fluid onto a hand of a user, and a fluid adjustor. The fluid adjustor is configured to automatically adjust a composition of the output fluid. The composition may be selected based on various considerations, such as: the health status of the user; the pH of the hand; the identity of the user; the history of the user; the schedule of the user; the predicted likelihood that the user has an infection; microbes detected on the hand; the moisture level of the hand; the concentration of one or more chemicals on the hand; an allergy profile of the user; and a chemical sensitivity profile of the user.

## Description

### Related Application

This application claims priority to the 23 February 2024 filing date of United States Provisional Patent Application Serial No. 63/557,293, which is incorporated herein by reference.

### Field of the Invention

This invention relates to hand cleaning fluid dispensers, and more particularly to dispensers in which two or more input substances are combined to generate an output fluid.

### Background of the Invention

Hand cleaning fluid dispensers are known in which the fluid that is dispensed is a mixture or combination of multiple chemical components, such as water, alcohol, and/or soap. The fluid is typically stored in a pre-mixed form in a fluid reservoir. Once the fluid reservoir is empty, the fluid reservoir can be replaced or refilled.

The applicant has appreciated a disadvantage of the prior art is that hand cleaning fluid dispensers are typically only able to dispense an output fluid having a static composition. Furthermore, prior art hand cleaning fluid dispensers typically need to be frequently serviced to refill or replace their fluid reservoirs.

### Summary of the Invention

To at least partially overcome some of the disadvantages of previously known systems, devices and methods, in one aspect the present invention provides a hand cleaning system comprising a hand cleaning fluid dispenser configured to dispense an output fluid onto a hand of a user, and a fluid adjustor. The fluid adjustor is preferably configured to automatically adjust a composition of the output fluid based, at least in part, on at least one of: a health status of the user; a predicted health status of the user; a pH of the hand; an identity of the user; a history of the user; a schedule of the user; a history of infections detected in a vicinity of the hand cleaning fluid dispenser; a predicted likelihood that the user has an infection; a microbiome of the hand; a time of day that the output fluid is dispensed; a date that the output fluid is dispensed; microbes detected on the hand; a moisture level of the hand; a concentration of one or more chemicals on the hand; an allergy profile of the user; and a chemical sensitivity profile of the user.

The applicant has appreciated that by adjusting the composition of the output fluid, the fluid can advantageously be tailored to the specific needs and/or preferences of the user. For example, if the user has an infection, the composition of the fluid can be adjusted to increase the biocidal activity of the fluid and reduce the risk of the user transmitting the infection. In some preferred embodiments, the system can detect specific pathogens present on the user's hand, and tailor the composition of the output fluid to target those specific pathogens.

Preferably, the hand cleaning fluid dispenser has two or more input reservoirs containing input substances that are combined to generate the output fluid. For example, one reservoir might contain a supply of water, and the other reservoir might contain a concentrated surfactant solution or a concentrated solvent. The fluid adjustor preferably adjusts the composition of the output fluid by adjusting the relative quantities of each of the input substances that are mixed together to generate the output fluid. For example, if the system determines that the user has an increased risk of transmitting an infection, the output fluid may be adjusted to have a higher concentration of surfactant or solvent.

Preferably, the system is able to draw water directly from the tap system in the facility where the fluid dispenser is installed. The tap system preferably provides a continuous supply of water that never needs to be manually refilled or replaced. Furthermore, since the water is preferably provided separately from the other input components, the other input components can preferably be stored in a highly concentrated form. This preferably allows the input reservoirs to last much longer before needing to be refilled or replaced as compared to traditional fluid reservoirs containing a pre-mixed hand cleaning fluid. Providing the input components in concentrated form also preferably reduces waste, pollution, transport costs, labor costs, and storage space requirements.

Further aspects of the invention include:
1. A hand cleaning system, which optionally includes one or more features of any one of more of the preceding and/or following aspects, comprising: a hand cleaning fluid dispenser configured to dispense an output fluid onto a hand of a user; and a fluid adjustor configured to automatically adjust a composition of the output fluid based, at least in part, on at least one of: a health status of the user; a predicted health status of the user; a pH of the hand; an identity of the user; a history of the user; a schedule of the user; a history of infections detected in a vicinity of the hand cleaning fluid dispenser; an infection profile for an area where the hand cleaning fluid dispenser is located; a predicted likelihood that the user has an infection; a microbiome of the hand; a time of day that the output fluid is dispensed; a date that the output fluid is dispensed; microbes detected on the hand; a moisture level of the hand; a concentration of one or more chemicals on the hand; an allergy profile of the user; and a chemical sensitivity profile of the user.
2. A hand cleaning system, which optionally includes one or more features of any one of more of the preceding and/or following aspects, wherein the fluid adjustor is configured to adjust the composition of the output fluid based, at least in part, on the health status of the user.
3. A hand cleaning system, which optionally includes one or more features of any one of more of the preceding and/or following aspects, wherein the fluid adjustor is configured to adjust the composition of the output fluid based, at least in part, on whether the user has an infection.
4. A hand cleaning system, which optionally includes one or more features of any one of more of the preceding and/or following aspects, wherein the fluid adjustor is configured to adjust the composition of the output fluid based, at least in part, on whether the user has an injury.
5. A hand cleaning system, which optionally includes one or more features of any one of more of the preceding and/or following aspects, wherein the fluid adjustor is configured to adjust the composition of the output fluid based, at least in part, on whether the user has an illness.
6. A hand cleaning system, which optionally includes one or more features of any one of more of the preceding and/or following aspects, wherein the fluid adjustor is configured to adjust the composition of the output fluid based, at least in part, on a body temperature of the user.
7. A hand cleaning system, which optionally includes one or more features of any one of more of the preceding and/or following aspects, wherein the fluid adjustor is configured to adjust the composition of the output fluid based, at least in part, on the predicted health status of the user.
8. A hand cleaning system, which optionally includes one or more features of any one of more of the preceding and/or following aspects, wherein the fluid adjustor is configured to adjust the composition of the output fluid based, at least in part, on the pH of the hand.
9. A hand cleaning system, which optionally includes one or more features of any one of more of the preceding and/or following aspects, wherein the fluid adjustor is configured to adjust the composition of the output fluid based, at least in part, on the identity of the user.
10. A hand cleaning system, which optionally includes one or more features of any one of more of the preceding and/or following aspects, wherein the fluid adjustor is configured to adjust the composition of the output fluid based, at least in part, on the history of the user.
11. A hand cleaning system, which optionally includes one or more features of any one of more of the preceding and/or following aspects, wherein the fluid adjustor is configured to adjust the composition of the output fluid based, at least in part, on a hand cleaning history of the user.
12. A hand cleaning system, which optionally includes one or more features of any one of more of the preceding and/or following aspects, wherein the fluid adjustor is configured to adjust the composition of the output fluid based, at least in part, on a history of activities performed by the user.
13. A hand cleaning system, which optionally includes one or more features of any one of more of the preceding and/or following aspects, wherein the fluid adjustor is configured to adjust the composition of the output fluid based, at least in part, on whether the user has engaged in an activity that increases a risk of the user carrying or transmitting an infectious agent.
14. A hand cleaning system, which optionally includes one or more features of any one of more of the preceding and/or following aspects, wherein the fluid adjustor is configured to adjust the composition of the output fluid based, at least in part, on whether the user has physically touched a person or an object that has been identified as being at risk of carrying an infectious agent.
15. A hand cleaning system, which optionally includes one or more features of any one of more of the preceding and/or following aspects, wherein the fluid adjustor is configured to adjust the composition of the output fluid based, at least in part, on whether the user has physically touched a patient in a healthcare facility.
16. A hand cleaning system, which optionally includes one or more features of any one of more of the preceding and/or following aspects, wherein the fluid adjustor is configured to adjust the composition of the output fluid based, at least in part, on whether the user has entered a patient room in a healthcare facility.
17. A hand cleaning system, which optionally includes one or more features of any one of more of the preceding and/or following aspects, wherein the fluid adjustor is configured to adjust the composition of the output fluid based, at least in part, on a history of locations that the user has travelled to.
18. A hand cleaning system, which optionally includes one or more features of any one of more of the preceding and/or following aspects, wherein the fluid adjustor is configured to adjust the composition of the output fluid based, at least in part, on whether the user has engaged in an activity that affects the pH of the hand.
19. A hand cleaning system, which optionally includes one or more features of any one of more of the preceding and/or following aspects, wherein the fluid adjustor is configured to adjust the composition of the output fluid based, at least in part, on the schedule of the user.
20. A hand cleaning system, which optionally includes one or more features of any one of more of the preceding and/or following aspects, wherein the fluid adjustor is configured to adjust the composition of the output fluid based, at least in part, on an upcoming activity to be performed by the user.
21. A hand cleaning system, which optionally includes one or more features of any one of more of the preceding and/or following aspects, wherein the fluid adjustor is configured to adjust the composition of the output fluid based, at least in part, on a location wherein the user is scheduled to travel to.
22. A hand cleaning system, which optionally includes one or more features of any one of more of the preceding and/or following aspects, wherein the fluid adjustor is configured to adjust the composition of the output fluid based, at least in part, on whether the user is about to enter a patient's room in a healthcare facility.
23. A hand cleaning system, which optionally includes one or more features of any one of more of the preceding and/or following aspects, wherein the fluid adjustor is configured to adjust the composition of the output fluid based, at least in part, on the history of infections detected in the vicinity of the hand cleaning fluid dispenser.
24. A hand cleaning system, which optionally includes one or more features of any one of more of the preceding and/or following aspects, wherein the fluid adjustor is configured to adjust the composition of the output fluid based, at least in part, on the predicted likelihood that the user has an infection.
25. A hand cleaning system, which optionally includes one or more features of any one of more of the preceding and/or following aspects, wherein the fluid adjustor is configured to adjust the composition of the output fluid based, at least in part, on the microbiome of the hand.
26. A hand cleaning system, which optionally includes one or more features of any one of more of the preceding and/or following aspects, wherein the fluid adjustor is configured to adjust the composition of the output fluid based, at least in part, on the time of day that the output fluid is dispensed.
27. A hand cleaning system, which optionally includes one or more features of any one of more of the preceding and/or following aspects, wherein the fluid adjustor is configured to adjust the composition of the output fluid based, at least in part, on the date that the output fluid is dispensed.
28. A hand cleaning system, which optionally includes one or more features of any one of more of the preceding and/or following aspects, wherein the fluid adjustor is configured to adjust the composition of the output fluid based, at least in part, on the microbes detected on the hand.
29. A hand cleaning system, which optionally includes one or more features of any one of more of the preceding and/or following aspects, wherein the fluid adjustor is configured to adjust the composition of the output fluid based, at least in part, on the microbes predicted to be on the hand.
30. A hand cleaning system, which optionally includes one or more features of any one of more of the preceding and/or following aspects, wherein the fluid adjustor is configured to adjust the composition of the output fluid based, at least in part, on the moisture level of the hand.
31. A hand cleaning system, which optionally includes one or more features of any one of more of the preceding and/or following aspects, wherein the fluid adjustor is configured to adjust the composition of the output fluid based, at least in part, on the concentration of one or more chemicals on the hand.
32. A hand cleaning system, which optionally includes one or more features of any one of more of the preceding and/or following aspects, wherein the fluid adjustor is configured to adjust the composition of the output fluid based, at least in part, on the concentration of fatty acids on the hand. The fatty acids preferably include short-chain fatty acids, but may also include long-chain fatty acids as well.
33. A hand cleaning system, which optionally includes one or more features of any one of more of the preceding and/or following aspects, wherein the fluid adjustor is configured to adjust the composition of the output fluid based, at least in part, on the allergy profile of the user.
34. A hand cleaning system, which optionally includes one or more features of any one of more of the preceding and/or following aspects, wherein the fluid adjustor is configured to adjust the composition of the output fluid based, at least in part, on the chemical sensitivity profile of the user.
35. A hand cleaning system, which optionally includes one or more features of any one of more of the preceding and/or following aspects, wherein the hand cleaning system further comprises at least one health status sensor.
36. A hand cleaning system, which optionally includes one or more features of any one of more of the preceding and/or following aspects, wherein the at least one health status sensor comprises a body temperature sensor.
37. A hand cleaning system, which optionally includes one or more features of any one of more of the preceding and/or following aspects, wherein the hand cleaning system further comprises a wearable device that is worn by the user; and wherein the at least one health status sensor comprises a wearable sensor that is incorporated into the wearable device.
38. A hand cleaning system, which optionally includes one or more features of any one of more of the preceding and/or following aspects, wherein the wearable device comprises a smart watch.
39. A hand cleaning system, which optionally includes one or more features of any one of more of the preceding and/or following aspects, wherein the at least one health status sensor comprises a camera.
40. A hand cleaning system, which optionally includes one or more features of any one of more of the preceding and/or following aspects, wherein the hand cleaning system further comprises a health status determination unit that is configured to determine the health status of the user based, at least in part, on data collected by the camera.
41. A hand cleaning system, which optionally includes one or more features of any one of more of the preceding and/or following aspects, wherein the health status determination unit is configured to interpret the data collected by the camera to recognize at least one of: coughing, sneezing, nose blowing, vomiting, bleeding, and frequency of bathroom use.
42. A hand cleaning system, which optionally includes one or more features of any one of more of the preceding and/or following aspects, wherein the hand cleaning system further comprises a health status prediction unit that is configured to predict the health status of the user based, at least in part, on data collected by the at least one health status sensor.
43. A hand cleaning system, which optionally includes one or more features of any one of more of the preceding and/or following aspects, wherein the hand cleaning system further comprises a pH determination device that is configured to determine the pH of the hand.
44. A hand cleaning system, which optionally includes one or more features of any one of more of the preceding and/or following aspects, wherein the pH determination device comprises a pH meter.
45. A hand cleaning system, which optionally includes one or more features of any one of more of the preceding and/or following aspects, wherein the pH meter is incorporated into the hand cleaning fluid dispenser.
46. A hand cleaning system, which optionally includes one or more features of any one of more of the preceding and/or following aspects, wherein the pH meter is carried by the user.
47. A hand cleaning system, which optionally includes one or more features of any one of more of the preceding and/or following aspects, wherein the pH determination device comprises a pH sensor that is incorporated into a smart phone.
48. A hand cleaning system, which optionally includes one or more features of any one of more of the preceding and/or following aspects, wherein the hand cleaning system further comprises a biometric identification device that is configured to determine the identity of the user based on biometric data.
49. A hand cleaning system, which optionally includes one or more features of any one of more of the preceding and/or following aspects, wherein the biometric identification device uses facial recognition to determine the identity of the user.
50. A hand cleaning system, which optionally includes one or more features of any one of more of the preceding and/or following aspects, wherein the hand cleaning system further comprises an electronic identification device that is configured to determine the identity of the user based on identification data received from a user identification device carried by the user.
51. A hand cleaning system, which optionally includes one or more features of any one of more of the preceding and/or following aspects, wherein the user identification device comprises at least one of: a smart watch, a smart phone, an identification tag, and an identification card.
52. A hand cleaning system, which optionally includes one or more features of any one of more of the preceding and/or following aspects, wherein the hand cleaning system further comprises a user history determination unit that is configured to determine the history of the user based, at least in part, on at least one of: location data for the user, dispenser use data for the user, activity data for the user, and human resource data for the user.
53. A hand cleaning system, which optionally includes one or more features of any one of more of the preceding and/or following aspects, wherein the hand cleaning system further comprises a location tracking device that tracks the location of the user over time.
54. A hand cleaning system, which optionally includes one or more features of any one of more of the preceding and/or following aspects, wherein the hand cleaning system further comprises a video recording and interpretation system that is configured to collect video data of the user and to identify activities performed by the user based, at least in part, on the video data.
55. A hand cleaning system, which optionally includes one or more features of any one of more of the preceding and/or following aspects, wherein the human resource data comprises a history of sick leave taken by the user.
56. A hand cleaning system, which optionally includes one or more features of any one of more of the preceding and/or following aspects, wherein the fluid adjustor is incorporated into the hand cleaning fluid dispenser.
57. A hand cleaning system, which optionally includes one or more features of any one of more of the preceding and/or following aspects, wherein the hand cleaning system further comprises a user schedule database that stores data about the schedule of the user.
58. A hand cleaning system, which optionally includes one or more features of any one of more of the preceding and/or following aspects, wherein the hand cleaning system is configured to collect data about the schedule of the user from a smart device carried by the user.
59. A hand cleaning system, which optionally includes one or more features of any one of more of the preceding and/or following aspects, wherein the hand cleaning system further comprises an infection history determination unit that determines the history of infections in the vicinity of the hand cleaning fluid dispenser based on infection data.
60. A hand cleaning system, which optionally includes one or more features of any one of more of the preceding and/or following aspects, wherein the hand cleaning system further comprises a network of infection detection devices, and wherein the infection data comprises data collected by the network of infection detection devices.
61. A hand cleaning system, which optionally includes one or more features of any one of more of the preceding and/or following aspects, wherein the infection data comprises public health data.
62. A hand cleaning system, which optionally includes one or more features of any one of more of the preceding and/or following aspects, wherein the hand cleaning system further comprises a microbe detection device that is configured to detect microbes on the hand.
63. A hand cleaning system, which optionally includes one or more features of any one of more of the preceding and/or following aspects, wherein the microbe detection device is configured to collect a sample from the hand, and analyze the sample.
64. A hand cleaning system, which optionally includes one or more features of any one of more of the preceding and/or following aspects, wherein the microbe detection device is configured to determine if the user has an infection.
65. A hand cleaning system, which optionally includes one or more features of any one of more of the preceding and/or following aspects, wherein the microbe detection device is configured to identify one or more microbes on the hand.
66. A hand cleaning system, which optionally includes one or more features of any one of more of the preceding and/or following aspects, wherein the microbe detection device is configured to characterize the microbiome of the hand.
67. A hand cleaning system, which optionally includes one or more features of any one of more of the preceding and/or following aspects, wherein the microbe detection device is incorporated into the hand cleaning fluid dispenser.
68. A hand cleaning system, which optionally includes one or more features of any one of more of the preceding and/or following aspects, wherein the hand cleaning system further comprises a time keeping device that monitors the time of day and the date that the output fluid is dispensed.
69. A hand cleaning system, which optionally includes one or more features of any one of more of the preceding and/or following aspects, wherein the hand cleaning system further comprises a moisture detecting device that is configured to detect the moisture level of the hand.
70. A hand cleaning system, which optionally includes one or more features of any one of more of the preceding and/or following aspects, wherein the moisture detecting device comprises a contactless moisture meter.
71. A hand cleaning system, which optionally includes one or more features of any one of more of the preceding and/or following aspects, wherein the moisture detecting device is incorporated into the hand cleaning fluid dispenser.
72. A hand cleaning system, which optionally includes one or more features of any one of more of the preceding and/or following aspects, wherein the hand cleaning system further comprises a chemical detecting device that is configured to detect one or more chemicals on the hand.
73. A hand cleaning system, which optionally includes one or more features of any one of more of the preceding and/or following aspects, wherein the chemical detecting device is configured to determine the concentration of fatty acids on the hand.
74. A hand cleaning system, which optionally includes one or more features of any one of more of the preceding and/or following aspects, wherein the hand cleaning system further comprises a user allergy database that stores data about the allergy profile of the user.
75. A hand cleaning system, which optionally includes one or more features of any one of more of the preceding and/or following aspects, wherein the hand cleaning system is configured to collect data about the allergy profile of the user from a smart device carried by the user.
76. A hand cleaning system, which optionally includes one or more features of any one of more of the preceding and/or following aspects, wherein the hand cleaning system further comprises a chemical sensitivity database that stores data about the chemical sensitivity profile of the user.
77. A hand cleaning system, which optionally includes one or more features of any one of more of the preceding and/or following aspects 5, wherein the hand cleaning system is configured to collect data about the chemical sensitivity profile of the user from a smart device carried by the user.
78. A hand cleaning system, which optionally includes one or more features of any one of more of the preceding and/or following aspects, wherein the fluid adjustor adjusts the composition of the output fluid by selecting a quantity of one or more input substances that are combined to form the output fluid.
79. A hand cleaning system, which optionally includes one or more features of any one of more of the preceding and/or following aspects, wherein the input substances comprise at least one of: water; a soap; an alcohol; a solvent; chlorine; a disinfectant solution; a surfactant; an acid; a base; a gas; sodium dodecyl sulfate; citric acid; acetic acid; sodium hydroxide; ozone; air; nitrogen; argon; carbon dioxide; a moisturizer; and glycerin.
80. A hand cleaning system, which optionally includes one or more features of any one of more of the preceding and/or following aspects, wherein the input substances comprise water.
81. A hand cleaning system, which optionally includes one or more features of any one of more of the preceding and/or following aspects, wherein the input substances comprise sodium dodecyl sulfate.
82. A hand cleaning system, which optionally includes one or more features of any one of more of the preceding and/or following aspects, wherein the input substances comprise citric acid.
83. A hand cleaning system, which optionally includes one or more features of any one of more of the preceding and/or following aspects, wherein the input substances comprise ozone.
84. A hand cleaning system, which optionally includes one or more features of any one of more of the preceding and/or following aspects, wherein the input substances comprise glycerin.
85. A hand cleaning system, which optionally includes one or more features of any one of more of the preceding and/or following aspects, wherein the input substances comprise a solution containing at least 10% sodium dodecyl sulfate by weight.
86. A hand cleaning system, which optionally includes one or more features of any one of more of the preceding and/or following aspects, wherein the input substances comprise a solution containing at least 10% citric acid by weight.
87. A hand cleaning system, which optionally includes one or more features of any one of more of the preceding and/or following aspects, wherein the hand cleaning system further comprises at least one mixing chamber for combining the input substances.
88. A hand cleaning system, which optionally includes one or more features of any one of more of the preceding and/or following aspects, wherein the hand cleaning system further comprises: a first input reservoir for supplying a first one of said input substances; and a second input reservoir for supplying a second one of said input substances.
89. A hand cleaning system, which optionally includes one or more features of any one of more of the preceding and/or following aspects, wherein the hand cleaning system further comprises a lock out feature that prevents the hand cleaning fluid dispenser from dispensing the output fluid when the first input reservoir is unable to supply the first one of said input substances.
90. A hand cleaning system, which optionally includes one or more features of any one of more of the preceding and/or following aspects, wherein the hand cleaning system further comprises a lock out feature that prevents the hand cleaning fluid dispenser from dispensing the output fluid when the second input reservoir is unable to supply the second one of said input substances.
91. A hand cleaning system, which optionally includes one or more features of any one of more of the preceding and/or following aspects, wherein the lock out feature also prevents the hand cleaning fluid dispenser from dispensing the output fluid when the second input reservoir is unable to supply the second one of said input substances.
92. A hand cleaning system, which optionally includes one or more features of any one of more of the preceding and/or following aspects, wherein the fluid adjustor is configured to: select a target pH value for the output fluid; and adjust the composition of the output fluid to so that the output fluid has the target pH value.
93. A hand cleaning system, which optionally includes one or more features of any one of more of the preceding and/or following aspects, where the target pH value is selected, at least in part, based on the pH of the hand.
94. A hand cleaning system, which optionally includes one or more features of any one of more of the preceding and/or following aspects, where the target pH value is selected, at least in part, so that the pH of the hand will fall within a target pH range after the output fluid is dispensed onto the hand.
95. A hand cleaning system, which optionally includes one or more features of any one of more of the preceding and/or following aspects, wherein the target pH range is between 4 and 5.
96. A hand cleaning system, which optionally includes one or more features of any one of more of the preceding and/or following aspects, wherein the target pH range is between 4.2 and 4.7.
97. A hand cleaning system, which optionally includes one or more features of any one of more of the preceding and/or following aspects, wherein the target pH range is between 4.2 and 4.5.
98. A hand cleaning system, which optionally includes one or more features of any one of more of the preceding and/or following aspects, where the target pH value is selected, at least in part, so that the pH of the hand will be about 4.3 after the output fluid is dispensed onto the hand.
99. A hand cleaning system, which optionally includes one or more features of any one of more of the preceding and/or following aspects, wherein the target pH value is selected, at least in part, to target a pathogen of concern.
100. A hand cleaning system, which optionally includes one or more features of any one of more of the preceding and/or following aspects, wherein the fluid adjustor is configured to adjust the composition of the output fluid to target a pathogen of concern.
101. A hand cleaning system, which optionally includes one or more features of any one of more of the preceding and/or following aspects, wherein the fluid adjustor is configured to adjust the composition of the output fluid to enhance an antimicrobial property of the output fluid when the user is determined to have an elevated risk of transmitting an infection.
102. A hand cleaning system, which optionally includes one or more features of any one of more of the preceding and/or following aspects, wherein the fluid adjustor is configured to adjust the composition of the output fluid to treat the injury.
103. A hand cleaning system, which optionally includes one or more features of any one of more of the preceding and/or following aspects, wherein the fluid adjustor is configured to adjust the composition of the output fluid to treat the illness.
104. A hand cleaning system, which optionally includes one or more features of any one of more of the preceding and/or following aspects, wherein the fluid adjustor is configured to adjust the composition of the output fluid based on a preference of the user.
105. A hand cleaning system, which optionally includes one or more features of any one of more of the preceding and/or following aspects, wherein the fluid adjustor is configured to adjust the composition of the output fluid based on a need of the user.
106. A hand cleaning system, which optionally includes one or more features of any one of more of the preceding and/or following aspects, wherein the fluid adjustor is configured to adjust the composition of the output fluid based on an occupational profile of the user.
107. A hand cleaning system, which optionally includes one or more features of any one of more of the preceding and/or following aspects, wherein the fluid adjustor is configured to adjust the composition of the output fluid based on an activity profile of the user.
108. A hand cleaning system, which optionally includes one or more features of any one of more of the preceding and/or following aspects, wherein the fluid adjustor is configured to adjust the composition of the output fluid to enhance an antimicrobial property of the output fluid when the user is determined to have an elevated risk of transmitting an infection to a vulnerable person.
109. A hand cleaning system, which optionally includes one or more features of any one of more of the preceding and/or following aspects, wherein the fluid adjustor is configured to adjust the composition of the output fluid to protect native microflora on the hand.
110. A hand cleaning system, which optionally includes one or more features of any one of more of the preceding and/or following aspects, wherein the fluid adjustor is configured to adjust the composition of the output fluid to target allochthonous microflora on the hand.
111. A hand cleaning system, which optionally includes one or more features of any one of more of the preceding and/or following aspects, wherein the fluid adjustor is configured to adjust the composition of the output fluid to moisturize the hand when the hand is determined to have a low moisture level.
112. A hand cleaning system, which optionally includes one or more features of any one of more of the preceding and/or following aspects, wherein the fluid adjustor is configured to adjust the composition of the output fluid to moisturize the hand when the user is determined to have an elevated risk of having dry hands.
113. A hand cleaning system, which optionally includes one or more features of any one of more of the preceding and/or following aspects, wherein the fluid adjustor is configured to adjust the composition of the output fluid to protect the hand from dryness when the user is determined to have an elevated risk of developing dry hands.
114. A hand cleaning system, which optionally includes one or more features of any one of more of the preceding and/or following aspects, wherein the fluid adjustor is configured to adjust the composition of the output fluid to protect the hand from dryness when the user is determined to engage in frequent hand cleaning.
115. A hand cleaning system, which optionally includes one or more features of any one of more of the preceding and/or following aspects, wherein the fluid adjustor is configured to adjust the composition of the output fluid to reduce or exclude a component that the user is allergic to.
116. A hand cleaning system, which optionally includes one or more features of any one of more of the preceding and/or following aspects, wherein the fluid adjustor is configured to adjust the composition of the output fluid to reduce or exclude a component that the user is sensitive to.
117. A hand cleaning system, which optionally includes one or more features of any one of more of the preceding and/or following aspects, wherein the output fluid has a pH between 3 and 5.
118. A hand cleaning system, which optionally includes one or more features of any one of more of the preceding and/or following aspects, wherein the output fluid has a pH between 3.5 and 4.5.
119. A hand cleaning system, which optionally includes one or more features of any one of more of the preceding and/or following aspects, wherein the output fluid has a pH of about 3.5.
120. A hand cleaning system, which optionally includes one or more features of any one of more of the preceding and/or following aspects, wherein the output fluid has a pH of about 4.
121. A hand cleaning system, which optionally includes one or more features of any one of more of the preceding and/or following aspects, wherein the output fluid has a pH of about 4.5.
122. A hand cleaning system, which optionally includes one or more features of any one of more of the preceding and/or following aspects, wherein the fluid adjustor is configured to increase a concentration of ozone in the output fluid when the hand is determined to be carrying Escherichia coli.
123. A hand cleaning system, which optionally includes one or more features of any one of more of the preceding and/or following aspects, wherein the fluid adjustor is configured to increase a concentration of glycerin in the output fluid when the hand is determined to be carrying Pseudomonas aeruginosa.
124. A hand cleaning fluid dispenser, which optionally includes one or more features of any one of more of the preceding and/or following aspects, comprising: a pump mechanism configured to dispense an output fluid onto a hand of a user; and a fluid adjustor configured to automatically adjust a composition of the output fluid based, at least in part, on at least one of: a health status of the user; a predicted health status of the user; a pH of the hand; an identity of the user; a history of the user; a schedule of the user; a history of infections detected in a vicinity of the hand cleaning fluid dispenser; a predicted likelihood that the user has an infection; a microbiome of the hand; a time of day that the output fluid is dispensed; a date that the output fluid is dispensed; microbes detected on the hand; a moisture level of the hand; a concentration of one or more chemicals on the hand; an allergy profile of the user; and a chemical sensitivity profile of the user.
125. A hand cleaning fluid dispenser, which optionally includes one or more features of any one of more of the preceding and/or following aspects, comprising: a water source; an input component reservoir that contains an input component; a mixing mechanism that combines at least the input component from the input component reservoir and water from the water source to generate an output fluid; and a pump mechanism that dispenses the output fluid onto a hand of a user.
126. A hand cleaning fluid dispenser, which optionally includes one or more features of any one of more of the preceding and/or following aspects, wherein the input component comprises a surfactant.
127. A hand cleaning fluid dispenser, which optionally includes one or more features of any one of more of the preceding and/or following aspects, wherein the input component comprises a soap.
128. A hand cleaning fluid dispenser, which optionally includes one or more features of any one of more of the preceding and/or following aspects, wherein the input component comprises an acid.
129. A hand cleaning fluid dispenser, which optionally includes one or more features of any one of more of the preceding and/or following aspects, wherein the input component comprises a gas.
130. A hand cleaning fluid dispenser, which optionally includes one or more features of any one of more of the preceding and/or following aspects, wherein the input component comprises a moisturizer.
131. A hand cleaning fluid dispenser, which optionally includes one or more features of any one of more of the preceding and/or following aspects, wherein the input component comprises sodium dodecyl sulfate.
132. A hand cleaning fluid dispenser, which optionally includes one or more features of any one of more of the preceding and/or following aspects, wherein the input component comprises citric acid.
133. A hand cleaning fluid dispenser, which optionally includes one or more features of any one of more of the preceding and/or following aspects, wherein the input component comprises ozone.
134. A hand cleaning fluid dispenser, which optionally includes one or more features of any one of more of the preceding and/or following aspects, wherein the input component comprises glycerin.
135. A hand cleaning fluid dispenser, which optionally includes one or more features of any one of more of the preceding and/or following aspects, wherein the hand cleaning fluid dispenser further comprises a foam generator; and wherein the output fluid is dispensed as a foam.
136. A hand cleaning fluid dispenser, which optionally includes one or more features of any one of more of the preceding and/or following aspects, wherein the input component comprises a solution containing at least 10% citric acid by weight.
137. A hand cleaning fluid dispenser, which optionally includes one or more features of any one of more of the preceding and/or following aspects, wherein the input component comprises a solution containing at least 10% sodium dodecyl sulfate by weight.
138. A hand cleaning fluid dispenser, which optionally includes one or more features of any one of more of the preceding and/or following aspects, wherein the output fluid comprises at least 0.5% sodium dodecyl sulfate by weight.
139. A hand cleaning fluid dispenser, which optionally includes one or more features of any one of more of the preceding and/or following aspects, wherein the output fluid comprises about 0.5% sodium dodecyl sulfate by weight.
140. A hand cleaning fluid dispenser, which optionally includes one or more features of any one of more of the preceding and/or following aspects, wherein the output fluid comprises about 1% sodium dodecyl sulfate by weight.
141. A hand cleaning fluid dispenser, which optionally includes one or more features of any one of more of the preceding and/or following aspects, wherein the output fluid comprises at least 0.5% citric acid by weight.
142. A hand cleaning fluid dispenser, which optionally includes one or more features of any one of more of the preceding and/or following aspects, wherein the output fluid comprises about 0.5% citric acid by weight.
143. A hand cleaning fluid dispenser, which optionally includes one or more features of any one of more of the preceding and/or following aspects, wherein the output fluid comprises about 1% citric acid by weight.
144. A hand cleaning fluid dispenser, which optionally includes one or more features of any one of more of the preceding and/or following aspects, wherein the output fluid comprises at least 1% glycerin by weight.
145. A hand cleaning fluid dispenser, which optionally includes one or more features of any one of more of the preceding and/or following aspects, wherein the output fluid comprises about 1% glycerin by weight.
146. A hand cleaning fluid dispenser, which optionally includes one or more features of any one of more of the preceding and/or following aspects, wherein the output fluid comprises about 10% glycerin by weight.
147. A hand cleaning fluid dispenser, which optionally includes one or more features of any one of more of the preceding and/or following aspects, wherein the output fluid comprises at least 500 ppm of ozone.
148. A hand cleaning fluid dispenser, which optionally includes one or more features of any one of more of the preceding and/or following aspects, wherein the output fluid has a pH between 3 and 5.
149. A hand cleaning fluid dispenser, which optionally includes one or more features of any one of more of the preceding and/or following aspects, wherein the output fluid has a pH between 3.5 and 4.7.
150. A hand cleaning fluid dispenser, which optionally includes one or more features of any one of more of the preceding and/or following aspects, wherein the output fluid has a pH between 4 and 4.5.
151. A hand cleaning fluid dispenser, which optionally includes one or more features of any one of more of the preceding and/or following aspects, wherein the output fluid has a pH of about 3.5.
152. A hand cleaning fluid dispenser, which optionally includes one or more features of any one of more of the preceding and/or following aspects, wherein the output fluid has a pH of about 4.
153. A hand cleaning fluid dispenser, which optionally includes one or more features of any one of more of the preceding and/or following aspects, wherein the output fluid has a pH of about 4.5.
154. A hand cleaning fluid dispenser, which optionally includes one or more features of any one of more of the preceding and/or following aspects, wherein the input component reservoir is a first input component reservoir, and the input component is a first input component; wherein the hand cleaning fluid dispenser further comprises a second input component reservoir containing a second input component; wherein the first input component comprises sodium dodecyl sulfate; wherein the second input component comprises citric acid; and wherein the mixing mechanism combines at least the first input component, the second input component, and the water to generate the output fluid.
155. A hand cleaning fluid dispenser, which optionally includes one or more features of any one of more of the preceding and/or following aspects, wherein the hand cleaning fluid dispenser further comprises a third input component reservoir containing a third input component; wherein the third input component comprises glycerin; and wherein the mixing mechanism combines at least the first input component, the second input component, the third input component, and the water to generate the output fluid.
156. A hand cleaning fluid dispenser, which optionally includes one or more features of any one of more of the preceding and/or following aspects, wherein the hand cleaning fluid dispenser further comprises an ozone generator for generating ozone; and wherein the mixing mechanism combines at least the first input component, the second input component, the third input component, the ozone, and the water to generate the output fluid.
157. A hand cleaning fluid dispenser, which optionally includes one or more features of any one of more of the preceding and/or following aspects, wherein the hand cleaning fluid dispenser further comprises a fluid adjustor configured to adjust a composition of the output fluid.
158. A hand cleaning fluid dispenser, which optionally includes one or more features of any one of more of the preceding and/or following aspects, wherein the fluid adjustor is configured to adjust the composition of the output fluid based on input from the user.
159. A hand cleaning fluid dispenser, which optionally includes one or more features of any one of more of the preceding and/or following aspects, wherein the fluid adjustor is configured to adjust the composition of the output fluid based on input from a facility administrator.
160. A hand cleaning fluid dispenser, which optionally includes one or more features of any one of more of the preceding and/or following aspects, wherein the fluid adjustor is configured to adjust the composition of the output fluid based, at least in part, on at least one of: a health status of the user; a predicted health status of the user; a pH of the hand; an identity of the user; a history of the user; a schedule of the user; a history of infections detected in a vicinity of the hand cleaning fluid dispenser; a predicted likelihood that the user has an infection; a microbiome of the hand; a time of day that the output fluid is dispensed; a date that the output fluid is dispensed; microbes detected on the hand; a moisture level of the hand; a concentration of one or more chemicals on the hand; an allergy profile of the user; and a chemical sensitivity profile of the user.
161. A hand cleaning fluid dispenser, which optionally includes one or more features of any one of more of the preceding and/or following aspects, wherein the fluid adjustor adjusts the composition of the output fluid by adjusting a quantity of the input component delivered to the mixing mechanism.
162. A hand cleaning fluid dispenser, which optionally includes one or more features of any one of more of the preceding and/or following aspects, wherein the fluid adjustor adjusts the composition of the output fluid by adjusting a quantity delivered to the mixing mechanism of at least one of: the input component; and the water.
163. A hand cleaning fluid dispenser, which optionally includes one or more features of any one of more of the preceding and/or following aspects, wherein the fluid adjustor adjusts the composition of the output fluid by adjusting a quantity delivered to the mixing mechanism of at least one of: the first input component; the second input component; and the water.
164. A hand cleaning fluid dispenser, which optionally includes one or more features of any one of more of the preceding and/or following aspects, wherein the fluid adjustor adjusts the composition of the output fluid by adjusting a quantity delivered to the mixing mechanism of at least one of: the first input component; the second input component; the third input component; and the water.
165. A hand cleaning fluid dispenser, which optionally includes one or more features of any one of more of the preceding and/or following aspects, wherein the fluid adjustor adjusts the composition of the output fluid by adjusting a quantity delivered to the mixing mechanism of at least one of: the first input component; the second input component; the third input component; the ozone; and the water.
166. A hand cleaning fluid dispenser, which optionally includes one or more features of any one of more of the preceding and/or following aspects, wherein the fluid adjustor adjusts the composition of the output fluid by adjusting a quantity of one or more input substances delivered to the mixing mechanism.
167. A hand cleaning fluid dispenser, which optionally includes one or more features of any one of more of the preceding and/or following aspects, wherein the mixing mechanism comprises at least one mixing chamber for combining at least some of the input substances.
168. A hand cleaning fluid dispenser, which optionally includes one or more features of any one of more of the preceding and/or following aspects, wherein the mixing mechanism comprises at least one mixing chamber for combining at least the input component and the water.
169. A hand cleaning fluid dispenser, which optionally includes one or more features of any one of more of the preceding and/or following aspects, wherein the mixing mechanism comprises at least one mixing chamber for combining at least the first input component, the second input component, and the water.
170. A hand cleaning fluid dispenser, which optionally includes one or more features of any one of more of the preceding and/or following aspects, wherein the mixing mechanism comprises at least one mixing chamber for combining at least the first input component, the second input component, the third input component, and the water.
171. A hand cleaning fluid dispenser, which optionally includes one or more features of any one of more of the preceding and/or following aspects, wherein the at least one mixing chamber is configured to deliver a premixed fluid to the pump mechanism.
172. A hand cleaning fluid dispenser, which optionally includes one or more features of any one of more of the preceding and/or following aspects, wherein the premixed fluid is the output fluid.
173. A hand cleaning fluid dispenser, which optionally includes one or more features of any one of more of the preceding and/or following aspects, further comprising a gas delivery mechanism that is configured to deliver a gas to the pump mechanism, wherein the output fluid comprises the premixed fluid combined with the gas.
174. A hand cleaning fluid dispenser, which optionally includes one or more features of any one of more of the preceding and/or following aspects, wherein the gas comprises ozone.
175. A hand cleaning fluid dispenser, which optionally includes one or more features of any one of more of the preceding and/or following aspects, wherein the at least one mixing chamber is configured to contain less than 20 mL of the premixed fluid.
176. A hand cleaning fluid dispenser, which optionally includes one or more features of any one of more of the preceding and/or following aspects, wherein the at least one mixing chamber is configured to contain about 15 mL of the premixed fluid.
177. A hand cleaning fluid dispenser, which optionally includes one or more features of any one of more of the preceding and/or following aspects, wherein the at least one mixing chamber is configured to discard the premixed fluid remaining in the at least one mixing chamber after the output fluid is dispensed onto the hand of the user.
178. A hand cleaning fluid, which optionally includes one or more features of any one of more of the preceding and/or following aspects, comprising water and sodium dodecyl sulfate.
179. A hand cleaning fluid, which optionally includes one or more features of any one of more of the preceding and/or following aspects, further comprising citric acid.
180. A hand cleaning fluid, which optionally includes one or more features of any one of more of the preceding and/or following aspects, further comprising glycerin.
181. A hand cleaning fluid, which optionally includes one or more features of any one of more of the preceding and/or following aspects, further comprising ozone.
182. A hand cleaning fluid, which optionally includes one or more features of any one of more of the preceding and/or following aspects, wherein the hand cleaning fluid is a foam.
183. A hand cleaning fluid, which optionally includes one or more features of any one of more of the preceding and/or following aspects, wherein the hand cleaning fluid comprises at least 0.5% sodium dodecyl sulfate by weight.
184. A hand cleaning fluid, which optionally includes one or more features of any one of more of the preceding and/or following aspects, wherein the hand cleaning fluid comprises about 0.5% sodium dodecyl sulfate by weight.
185. A hand cleaning fluid, which optionally includes one or more features of any one of more of the preceding and/or following aspects, wherein the hand cleaning fluid comprises about 1% sodium dodecyl sulfate by weight.
186. A hand cleaning fluid, which optionally includes one or more features of any one of more of the preceding and/or following aspects, wherein the hand cleaning fluid comprises at least 0.5% citric acid by weight.
187. A hand cleaning fluid, which optionally includes one or more features of any one of more of the preceding and/or following aspects, wherein the hand cleaning fluid comprises about 0.5% citric acid by weight.
188. A hand cleaning fluid, which optionally includes one or more features of any one of more of the preceding and/or following aspects, wherein the hand cleaning fluid comprises about 1% citric acid by weight.
189. A hand cleaning fluid, which optionally includes one or more features of any one of more of the preceding and/or following aspects, wherein the hand cleaning fluid comprises at least 1% glycerin by weight.
190. A hand cleaning fluid, which optionally includes one or more features of any one of more of the preceding and/or following aspects, wherein the hand cleaning fluid comprises about 1% glycerin by weight.
191. A hand cleaning fluid, which optionally includes one or more features of any one of more of the preceding and/or following aspects, wherein the hand cleaning fluid comprises about 10% glycerin by weight.
192. A hand cleaning fluid, which optionally includes one or more features of any one of more of the preceding and/or following aspects, wherein the hand cleaning fluid comprises at least 500 ppm of ozone.
193. A hand cleaning fluid, which optionally includes one or more features of any one of more of the preceding and/or following aspects, wherein the hand cleaning fluid has a pH between 3 and 5.
194. A hand cleaning fluid, which optionally includes one or more features of any one of more of the preceding and/or following aspects, wherein the hand cleaning fluid has a pH between 3.5 and 4.7.
195. A hand cleaning fluid, which optionally includes one or more features of any one of more of the preceding and/or following aspects, wherein the hand cleaning fluid has a pH between 4 and 4.5.
196. A hand cleaning fluid, which optionally includes one or more features of any one of more of the preceding and/or following aspects, wherein the hand cleaning fluid has a pH of about 3.5.
197. A hand cleaning fluid, which optionally includes one or more features of any one of more of the preceding and/or following aspects, wherein the hand cleaning fluid has a pH of about 4.
198. A hand cleaning fluid, which optionally includes one or more features of any one of more of the preceding and/or following aspects, wherein the hand cleaning fluid has a pH of about 4.5.
199. A method, which optionally includes one or more features of any one of more of the preceding and/or following aspects comprising: configuring a hand cleaning fluid dispenser to automatically adjust a composition of a hand cleaning fluid dispensed onto a hand of a user based, at least in part, on at least one of: a health status of the user; a predicted health status of the user; a pH of the hand; an identity of the user; a history of the user; a schedule of the user; a history of infections detected in a vicinity of the hand cleaning fluid dispenser; a predicted likelihood that the user has an infection; a microbiome of the hand; a time of day that the output fluid is dispensed; a date that the output fluid is dispensed; microbes detected on the hand; a moisture level of the hand; a concentration of one or more chemicals on the hand; an allergy profile of the user; and a chemical sensitivity profile of the user.
200. A hand cleaning system, which optionally includes one or more features of any one of more of the preceding and/or following aspects, comprising: a hand cleaning fluid dispenser configured to dispense an output fluid onto a hand of a user; and a fluid adjustor configured to adjust a composition of the output fluid based on a characteristic and/or an identity of the user.
201. A hand cleaning system, which optionally includes one or more features of any one of more of the preceding and/or following aspects, comprising: a hand cleaning fluid dispenser configured to dispense an output fluid onto a hand of a user; and a fluid adjustor configured to adjust a composition of the output fluid based on a characteristic of the user.
202. A hand cleaning system, which optionally includes one or more features of any one of more of the preceding and/or following aspects, comprising: a hand cleaning fluid dispenser configured to dispense an output fluid onto a hand of a user; and a fluid adjustor configured to adjust a composition of the output fluid based on an identity of the user.
203. A hand cleaning system, a hand cleaning fluid dispenser, or a method, which optionally includes one or more features of any one of more of the preceding and/or following aspects, wherein the input component comprises a solvent, preferably an alcohol or a disinfectant solution.
204. A hand cleaning system, a hand cleaning fluid dispenser, or a method, which optionally includes one or more features of any one of more of the preceding and/or following aspects, wherein the fluid adjustor is configured to adjust the composition of the output fluid based, at least in part, on the infection profile for the area where the hand cleaning fluid dispenser is located.
205. A hand cleaning system, a hand cleaning fluid dispenser, or a method, which optionally includes one or more features of any one of more of the preceding and/or following aspects, wherein the infection profile is based, at least in part, on data about pathogens that have been identified or are predicted to be circulating in the area where the hand cleaning fluid dispenser is located.
206. A hand cleaning system, a hand cleaning fluid dispenser, or a method, which optionally includes one or more features of any one of more of the preceding and/or following aspects, wherein the fluid adjustor is configured to increase a concentration of ozone in the output fluid when the hand is determined to be carrying any kind of pathogen or transient bacteria.
207. A hand cleaning system, a hand cleaning fluid dispenser, or a method, which optionally includes one or more features of any one of more of the preceding and/or following aspects, wherein the fluid adjustor is configured to increase a concentration of glycerin in the output fluid when the hand is determined to be carrying Staphylococcus aureus.
208. A hand cleaning system, a hand cleaning fluid dispenser, or a method, which optionally includes one or more features of any one of more of the preceding and/or following aspects, wherein the fluid adjustor is configured to increase a concentration of glycerin in the output fluid when the hand is determined to be carrying Methicillin-resistant Staphylococcus aureus.
209. A hand cleaning system, a hand cleaning fluid dispenser, or a method, which optionally includes one or more features of any one of more of the preceding and/or following aspects, wherein the input component comprises a chemical solvent.
210. A hand cleaning system, a hand cleaning fluid dispenser, or a method, which optionally includes one or more features of any one of more of the preceding and/or following aspects, wherein the input component comprises an alcohol.
211. A hand cleaning system, a hand cleaning fluid dispenser, or a method, which optionally includes one or more features of any one of more of the preceding and/or following aspects, wherein the input component comprises ethanol.
212. A hand cleaning system, a hand cleaning fluid dispenser, or a method, which optionally includes one or more features of any one of more of the preceding and/or following aspects, wherein the input component comprises isopropanol.
213. A hand cleaning system, a hand cleaning fluid dispenser, or a method, which optionally includes one or more features of any one of more of the preceding and/or following aspects, wherein the fluid adjustor adjusts the composition of the output fluid by adjusting a quantity delivered to the mixing mechanism of at least one of: the first input component; the second input component; the third input component; any gaseous components; and the water.
214. A hand cleaning system, a hand cleaning fluid dispenser, or a method, which optionally includes one or more features of any one of more of the preceding and/or following aspects, further comprising a gas delivery mechanism that is configured to deliver any gaseous components to the pump mechanism, wherein the output fluid comprises the premixed fluid combined with the gaseous components.
215. A hand cleaning system, a hand cleaning fluid dispenser, or a method, which optionally includes one or more features of any one of more of the preceding and/or following aspects, wherein the gaseous components comprise carbon dioxide and/or ozone.
216. A hand cleaning system, which optionally includes one or more features of any one of more of the preceding and/or following aspects, comprising: a hand cleaning fluid dispenser configured to dispense an output fluid onto a hand of a user; and a fluid adjustor configured to automatically adjust a composition of the output fluid based, at least in part, on: a health status of the user; a predicted health status of the user; a pH of the hand; an identity of the user; a history of the user; a schedule of the user; a history of infections detected in a vicinity of the hand cleaning fluid dispenser; an infection profile for an area where the hand cleaning fluid dispenser is located; a predicted likelihood that the user has an infection; a microbiome of the hand; a time of day that the output fluid is dispensed; a date that the output fluid is dispensed; microbes detected on the hand; a moisture level of the hand; a concentration of one or more chemicals on the hand; an allergy profile of the user; or a chemical sensitivity profile of the user.
217. A hand cleaning system, which optionally includes one or more features of any one of more of the preceding and/or following aspects, wherein the hand cleaning system further comprises at least one health status sensor.
218. A hand cleaning system, which optionally includes one or more features of any one of more of the preceding and/or following aspects, wherein the at least one health status sensor comprises at least one of: a body temperature sensor; a wearable sensor that is incorporated into a wearable device; and a camera.
219. A hand cleaning system, which optionally includes one or more features of any one of more of the preceding and/or following aspects, wherein the hand cleaning system further comprises a health status determination unit that is configured to determine the health status of the user based, at least in part, on data collected by the camera; and wherein the health status determination unit is configured to interpret the data collected by the camera to recognize at least one of: coughing, sneezing, nose blowing, vomiting, bleeding, and frequency of bathroom use.
220. A hand cleaning system, which optionally includes one or more features of any one of more of the preceding and/or following aspects, wherein the hand cleaning system further comprises a pH determination device that is configured to determine the pH of the hand.
221. A hand cleaning system, which optionally includes one or more features of any one of more of the preceding and/or following aspects, wherein the pH determination device comprises at least one of: a pH meter; a pH meter that is incorporated into the hand cleaning fluid dispenser; a pH meter that is carried by the user; and a pH sensor that is incorporated into a smart phone.
222. A hand cleaning system, which optionally includes one or more features of any one of more of the preceding and/or following aspects, wherein the hand cleaning system further comprises a biometric identification device that is configured to determine the identity of the user based on biometric data.
223. A hand cleaning system, which optionally includes one or more features of any one of more of the preceding and/or following aspects, wherein the hand cleaning system further comprises an electronic identification device that is configured to determine the identity of the user based on identification data received from a user identification device carried by the user; and wherein the user identification device comprises at least one of: a smart watch, a smart phone, an identification tag, and an identification card.
224. A hand cleaning system, which optionally includes one or more features of any one of more of the preceding and/or following aspects, wherein the hand cleaning system further comprises a user history determination unit that is configured to determine the history of the user based, at least in part, on at least one of: location data for the user, dispenser use data for the user, activity data for the user, and human resource data for the user.
225. A hand cleaning system, which optionally includes one or more features of any one of more of the preceding and/or following aspects, wherein the hand cleaning system further comprises a video recording and interpretation system that is configured to collect video data of the user and to identify activities performed by the user based, at least in part, on the video data.
226. A hand cleaning system, which optionally includes one or more features of any one of more of the preceding and/or following aspects, wherein the fluid adjustor is incorporated into the hand cleaning fluid dispenser.
227. A hand cleaning system, which optionally includes one or more features of any one of more of the preceding and/or following aspects, wherein the hand cleaning system further comprises a microbe detection device that is configured to detect microbes on the hand.
228. A hand cleaning system, which optionally includes one or more features of any one of more of the preceding and/or following aspects, wherein the hand cleaning system further comprises a moisture detecting device that is configured to detect the moisture level of the hand; wherein the moisture detecting device comprises a contactless moisture meter; and wherein the moisture detecting device is incorporated into the hand cleaning fluid dispenser.
229. A hand cleaning system, which optionally includes one or more features of any one of more of the preceding and/or following aspects, wherein the hand cleaning system further comprises a chemical detecting device that is configured to detect one or more chemicals on the hand; and wherein the chemical detecting device is configured to determine the concentration of fatty acids on the hand.
230. A hand cleaning system, which optionally includes one or more features of any one of more of the preceding and/or following aspects, wherein the fluid adjustor adjusts the composition of the output fluid by selecting a quantity of one or more input substances that are combined to form the output fluid; wherein the input substances comprise at least one of: water; a soap; an alcohol; a solvent; a disinfectant solution; chlorine; a surfactant; an acid; a base; a gas; sodium dodecyl sulfate; citric acid; acetic acid; sodium hydroxide; ozone; air; nitrogen; argon; carbon dioxide; a moisturizer; and glycerin; wherein the hand cleaning system further comprises at least one mixing chamber for combining the input substances.
231. A hand cleaning system, which optionally includes one or more features of any one of more of the preceding and/or following aspects, wherein the hand cleaning system further comprises: a first input reservoir for supplying a first one of said input substances; a second input reservoir for supplying a second one of said input substances; and a lock out feature that prevents the hand cleaning fluid dispenser from dispensing the output fluid when the first input reservoir is unable to supply the first one of said input substances.
232. A hand cleaning fluid dispenser, which optionally includes one or more features of any one of more of the preceding and/or following aspects, comprising: a pump mechanism configured to dispense an output fluid onto a hand of a user; and a fluid adjustor configured to automatically adjust a composition of the output fluid based, at least in part, on: a health status of the user; a predicted health status of the user; a pH of the hand; an identity of the user; a history of the user; a schedule of the user; a history of infections detected in a vicinity of the hand cleaning fluid dispenser; an infection profile for an area where the hand cleaning fluid dispenser is located; a predicted likelihood that the user has an infection; a microbiome of the hand; a time of day that the output fluid is dispensed; a date that the output fluid is dispensed; microbes detected on the hand; a moisture level of the hand; a concentration of one or more chemicals on the hand; an allergy profile of the user; or a chemical sensitivity profile of the user.
233. A hand cleaning fluid dispenser, which optionally includes one or more features of any one of more of the preceding and/or following aspects, comprising: a water source; an input component reservoir that contains an input component; a mixing mechanism that combines at least the input component from the input component reservoir and water from the water source to generate an output fluid; and a pump mechanism that dispenses the output fluid onto a hand of a user.
234. A hand cleaning fluid, which optionally includes one or more features of any one of more of the preceding and/or following aspects, comprising water and sodium dodecyl sulfate.
235. A method, which optionally includes one or more features of any one of more of the preceding and/or following aspects, comprising: configuring a hand cleaning fluid dispenser to automatically adjust a composition of a hand cleaning fluid dispensed onto a hand of a user based, at least in part, on: a health status of the user; a predicted health status of the user; a pH of the hand; an identity of the user; a history of the user; a schedule of the user; a history of infections detected in a vicinity of the hand cleaning fluid dispenser; an infection profile for an area where the hand cleaning fluid dispenser is located; a predicted likelihood that the user has an infection; a microbiome of the hand; a time of day that the output fluid is dispensed; a date that the output fluid is dispensed; microbes detected on the hand; a moisture level of the hand; a concentration of one or more chemicals on the hand; an allergy profile of the user; or a chemical sensitivity profile of the user.

### Brief Description of the Drawings

Further aspects and advantages of the invention will appear from the following description taken together with the accompanying drawings, in which:
Figure 1 is a front view of a fluid dispenser in accordance with a first embodiment of the present invention;
Figure 2 is a schematic representation of a hand cleaning system incorporating the fluid dispenser shown in Figure 1;
Figure 3 is a perspective view of a fluid dispenser in accordance with a second embodiment of the present invention; and
Figure 4 is a graph showing the results of an experiment investigating the relationship between skin pH and the killing rate of hand cleaning solutions.

### Detailed Description of the Drawings

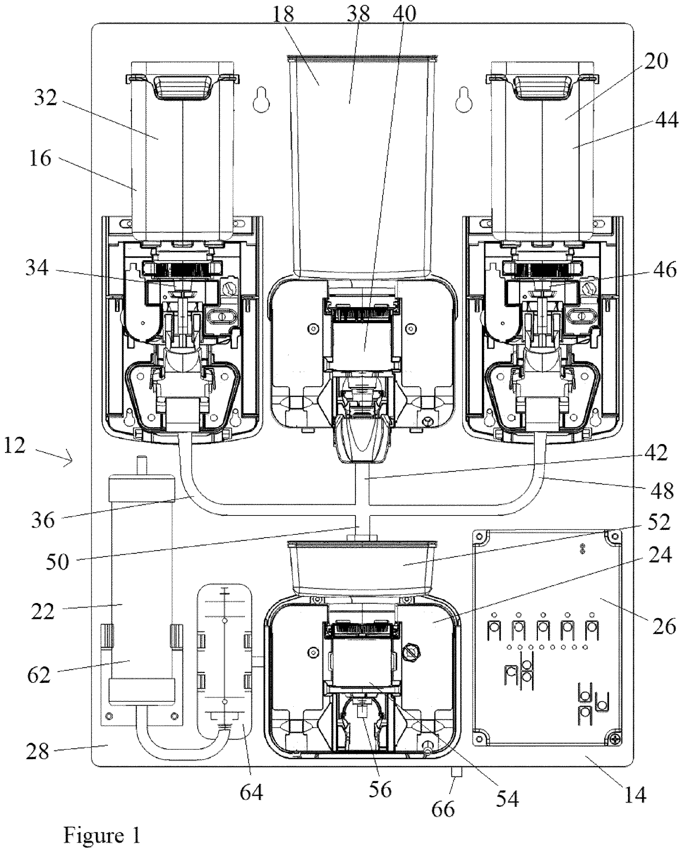

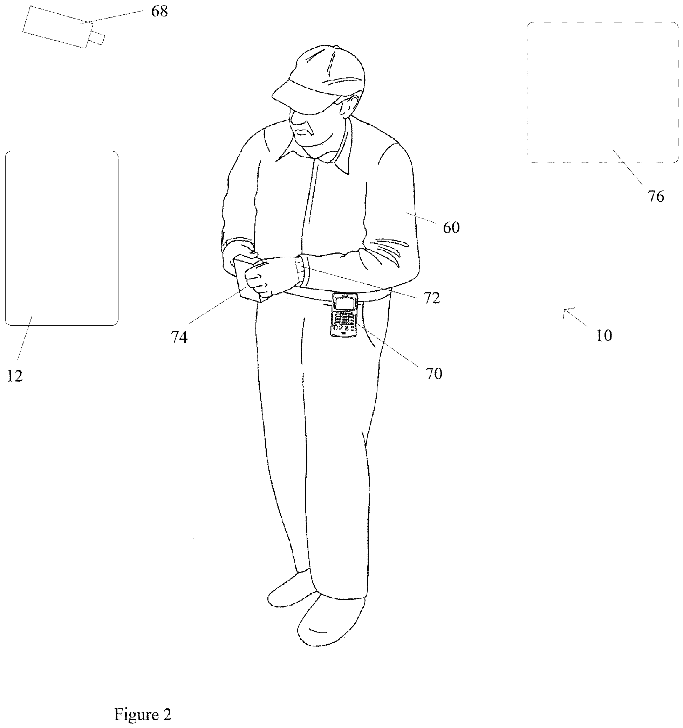

Figures 1 and 2 show a hand cleaning system 10 and a hand cleaning fluid dispenser 12 in accordance with a first embodiment of the present invention. As can be seen in Figure 1, the hand cleaning fluid dispenser 12 includes a housing 14, a first input dispenser 16, a second input dispenser 18, a third input dispenser 20, an ozone generator 22, an output dispenser 24, and a controller 26.

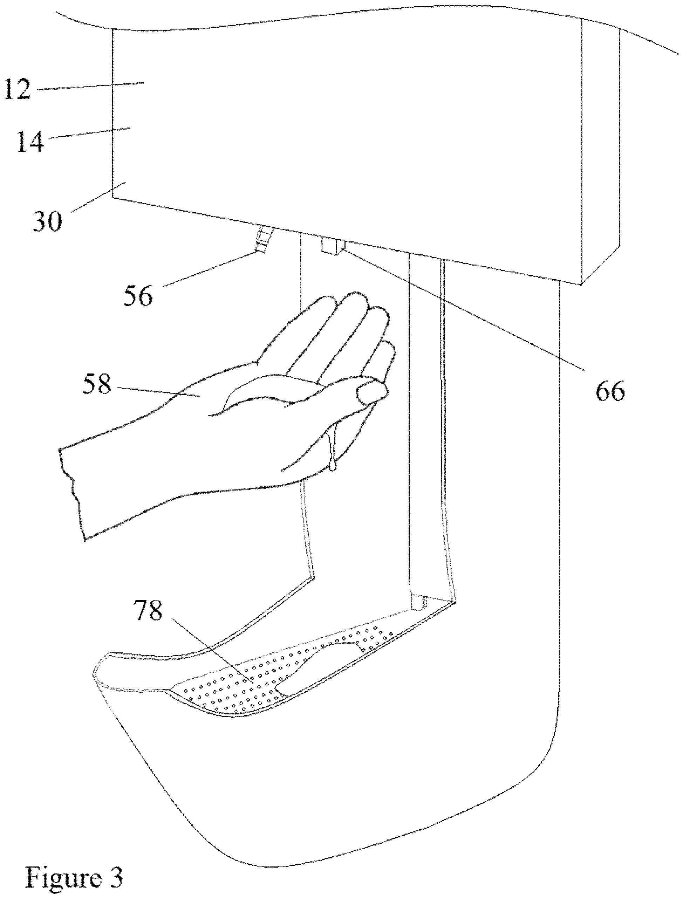

The housing 14 includes a back plate 28 that is configured to be mounted to a vertical support such as a wall or a post. The back plate 28 carries the first input dispenser 16, the second input dispenser 18, the third input dispenser 20, the ozone generator 22, the output dispenser 24, and the controller 26. Although not shown in Figure 1, the housing 14 preferably includes a removable cover 30 that attaches to the back plate 28 to cover the first input dispenser 16, the second input dispenser 18, the third input dispenser 20, the ozone generator 22, the output dispenser 24, and the controller 26. An example of a removable cover 30 can be seen in Figure 3.

The first input dispenser 16 includes a first input reservoir 32 that stores a first input fluid, and a first pump mechanism 34 for delivering the first input fluid to a first input channel 36. The second input dispenser 18 similarly includes a second input reservoir 38 that stores a second input fluid, and a second pump mechanism 40 for delivering the second input fluid to a second input channel 42. The third input dispenser 20 likewise includes a third input reservoir 44 that stores a third input fluid, and a third pump mechanism 46 for delivering the third input fluid to a third input channel 48. The first input channel 36, the second input channel 42, and the third input channel 48 converge to form a unified input channel 50 for delivering the first, second, and third input fluids to the output dispenser 24.

The first input reservoir 32, the second input reservoir 38, and the third input reservoir 44 may contain any suitable fluids that can be combined to form a hand cleaning fluid to be output from the dispenser 12. Preferably, one of the reservoirs 38 provides a supply of water, and the other two reservoirs 32, 44 contain concentrated input substances. For example, in one preferred embodiment the first input reservoir 32 contains a concentrated sodium dodecyl sulfate solution, the second input reservoir 38 contains water, and the third input reservoir 44 contains a concentrated citric acid solution. The second input reservoir 38 is preferably connected to the tap water system in the facility where the dispenser 12 is located, and automatically refills with water whenever the water level in the reservoir 38 is running low.

The first input dispenser 16, the second input dispenser 18, and the third input dispenser 20 may have any suitable structures for delivering the first, second, and third input fluids to the output dispenser 24. For example, the first input dispenser 16, the second input dispenser 18, and the third input dispenser 20 may incorporate any structure or combination of structures disclosed in any one or more of the following: United States Patent No. 7,748,573 to Anhuf et al., issued July 6, 2010; U.S. Patent No. 5,975,360 to Ophardt, issued November 2, 1999; U.S. 7,984,825 to Ophardt et al., issued July 26, 2011; U.S. 8,397,949 to Ophardt, issued March 19, 2013; U.S. 9,027,788 to Ophardt et al., issued May 12, 2015; U.S. 8,622,243 to Ophardt et al., issued January 7, 2014; U.S. 8,733,596 to Ophardt et al., issued May 27, 2004; U.S. 7,455,197 to Ophardt, issued November 25, 2008; United States Patent No. 8,245,877 to Ophardt, issued August 21, 2012; United States Patent No. 8,113,388 to Ophardt et al., issued February 14, 2012; United States Patent No. 8,091,739 to Ophardt et al., issued January 10, 2012; U.S. Patent No. 8,684,236 to Ophardt, issued April 1, 2014; U.S. Patent No. 5,373,970 to Ophardt, issued December 20, 1994; U.S. Patent No. 5,836,482 to Ophardt et al., issued November 17, 1998; U.S. Patent No. 9,682,390 to Ophardt et al., issued June 20, 2017; United States Patent No. 10,242,301 to Ophardt et al., issued March 26, 2019; United States Patent No. 8,413,852 to Ophardt et al., issued April 9, 2013; United States Patent No. 8,113,388 to Ophardt et al., issued February 14, 2012; and U.S. 7,455,197 to Ophardt, issued November 25, 2008, which are incorporated herein by reference.

The output dispenser 24 includes a mixing chamber 52, an output pump mechanism 54 and a fluid outlet 56. The mixing chamber 52 receives the first input fluid, the second input fluid, and the third input fluid from the unified input channel 50. The first input fluid, the second input fluid, and the third input fluid are mixed together in the mixing chamber 52 to form a premixed fluid. Optionally, the mixing chamber 52 includes a mixing device such as a magnetic stir bar to enhance the mixing of the input fluids.

The output pump mechanism 54 is preferably configured to mix the premixed fluid with a gas to generate a foam, which is then dispensed from the fluid outlet 56 onto the hand 58 of a user 60. The output pump mechanism 54 may incorporate any suitable mechanisms for generating foam, including any one or more of the structures disclosed in any one or more of the following: United States Patent No. 8,733,596 to Ophardt et al., issued May 27, 2014; U.S. Patent No. 7,303,099 to Ophardt, issued December 4, 2007; U.S. Patent No. 8,272,539 to Ophardt et al., issued September 25, 2012; U.S. Patent No. 8,733,596 to Ophardt et al., issued May 27, 2014; U.S. Patent No. 9,573,152 to Ophardt et al., issued February 21, 2017; and U.S. Patent No. 10,105,018 to Jones et al., issued October 23, 2018, which are incorporated herein by reference.

Preferably, the gas that is used to generate the foam is drawn from the ozone generator 22. The ozone generator 22 includes a desiccant chamber 62 and an ozone generation chamber 64. The ozone generator 22 preferably draws air from the atmosphere, which passes through the desiccant chamber 62 to remove moisture from the air. The air then passes through the ozone generation chamber 64, which converts oxygen in the air into ozone. Any suitable method of generating ozone could be used, including a corona discharge and/or UV radiation. Any suitable structure for generating ozone and delivering the ozone to the output pump mechanism 54 could be used, including any of the structures disclosed in any one or more of the following: United States Patent No. 8,733,596 to Ophardt et al., issued May 27, 2014; U.S. Patent No. 7,303,099 to Ophardt, issued December 4, 2007; U.S. Patent No. 8,272,539 to Ophardt et al., issued September 25, 2012; U.S. Patent No. 8,733,596 to Ophardt et al., issued May 27, 2014; U.S. Patent No. 9,573,152 to Ophardt et al., issued February 21, 2017; U.S. Patent No. 10,105,018 to Jones et al., issued October 23, 2018; U.S. Publication No. 2021/0244756 to Ophardt, published 12 August 2021; and U.S. Publication No. 2021/0106182 to Ophardt et al., published 15 April 2021, which are incorporated herein by reference.

The controller 26 is configured to control the operation of the first input dispenser 16, the second input dispenser 18, the third input dispenser 20, the ozone generator 22, and the output dispenser 24. The controller 26 preferably includes one or more processors, one or more wireless communication devices, one or more memories, and a power source. Preferably, the controller 26 functions as a fluid adjustor, in that it controls the operations of the first input dispenser 16, the second input dispenser 18, the third input dispenser 20, and the ozone generator 22 in order to adjust the composition of the fluid that is output from the fluid outlet 56. For example, the controller 26 may control the first input dispenser 16 to reduce the volume of the first input fluid that is delivered to the mixing chamber 52, and thereby reduce the concentration of sodium dodecyl sulfate in the output fluid. The controller 26 can similarly increase, reduce, and/or eliminate the delivery of the second input fluid and/or the third input fluid to the mixing chamber 52. The controller 26 can also control the function of the ozone generator 22 in order to adjust the concentration of ozone in the output foam. For example, the activation time of the ozone generator 22 could be increased to increase the concentration of ozone, or could be decreased to decrease the concentration of ozone. The ozone generator 22 could also be inactivated, so that the gas delivered to the output dispenser 24 is air without any added ozone.

Preferably, the hand cleaning fluid dispenser 12 incorporates at least one sensor 66 that is configured to collect information about the user 60. This information is then preferably used by the controller 26 to determine the composition of the output fluid.

For example, the at least one sensor 66 could include a touchless temperature sensor that is configured to detect the body temperature of the user 60. The controller 66 can furthermore be programmed to assess the body temperature of the user 60 in order to determine whether the user 60 likely has an infection, and to adjust the composition of the output fluid accordingly. For example, if the controller 26 determines that the user 60 likely has an infection, the concentration of sodium dodecyl sulfate and/or ozone could be increased, in order to increase the biocidal properties of the output fluid. Alternatively, if the controller 26 determines that the user 60 likely does not have an infection, the concentration of sodium dodecyl sulfate and/or ozone could be decreased, in order to reduce irritation to the user's skin.

The at least one sensor 66 could also include an optical sensor that is configured to detect one or more optical properties of the user's skin. For example, the optical sensor could be configured to detect redness, indicating skin irritation or an injury. The controller 26 could then adjust the composition of the output fluid, for example to decrease the concentration of components known to cause skin irritation and/or to increase the concentration of components known to soothe irritation and/or promote healing.

The at least one sensor 66 could also include a pH sensor that is configured to detect the pH of the skin on the user's hand 58. The pH sensor could, for example, be in the form of a pH meter that detects pH by measuring the electrical potential of the user's skin. As will be described in more detail below, the applicant has found that the pH of the user's skin can have important implications for the effectiveness of a hand cleaning fluid. The controller 26 can accordingly adjust the composition of the output fluid based on the pH measurement in order to maximize the effectiveness of the hand cleaning fluid and/or reduce skin irritation.

The at least one sensor 66 could also include a moisture sensor that senses the moisture level of the user's hand 58. The controller 26 can then use the moisture data in order to determine whether the user's hand 58 is excessively dry and/or is at risk of becoming excessively dry, and adjust the composition of the output fluid accordingly. For example, if the user's hand 58 is determined to be excessively dry or at risk of becoming excessively dry, the composition of the output fluid can be adjusted to increase the concentration of components known to moisturize the hand 58 and/or to reduce the concentration of components known to increase dryness.

Optionally, the hand cleaning system 10 includes additional components that are not incorporated directly into the hand cleaning fluid dispenser 12. For example, as shown in Figure 2, the system 10 could also include one or more of the following: a camera 68, a smartphone 70 carried by the user 60, a smart watch 72 worn by the user 60, a handheld sensor device 74 carried by the user, and/or an external server 76.

The camera 68 may, for example, be placed in proximity to the fluid dispenser 12 in order to capture images of the user 60 when the user 60 approaches the dispenser 12. The images can then be processed by the controller 26 and/or the external server 76 in order to identify the user 60, for example using facial recognition software. Optionally, the controller 26 and/or the external server 76 stores hand cleaning profiles for one or more regular users 60 of the dispenser 12, and the controller 26 selects the appropriate profile for the user 60 based on the facial recognition data.

The hand cleaning profile preferably includes information about the hand cleaning preferences and/or needs of the user 60, which are used by the controller 26 when determining the composition of the output fluid. For example, the user profile may indicate that the user 60 has a chemical sensitivity or is allergic to a particular component, and in response the controller 26 may be configured to reduce or eliminate that component from the output fluid.

The user profile might also include information such as the hand cleaning habits of the user 60. If the user 60 engages in frequent hand cleaning, the controller 26 may be configured to reduce the concentration of components known to cause skin irritation when used frequently. If the user 60 washes their hands infrequently, the controller 26 may increase the concentration of biocidal components in order to maximize the effectiveness of the output fluid.

In some embodiments of the invention, the system 10 may use data collected and/or provided by one or more of the smartphone 70, the smart watch 72, or the handheld sensor device 74 to determine the composition of the output fluid. For example, the smart watch 74 may incorporate wearable sensors that collect information about the user 60, such as location history, heart rate, body temperature, blood oxygen levels, and/or perspiration. The system 10 may take this information into account in determining whether the user 60 is likely to have an infection, or is at risk transmitting a pathogen. For example, if the location history data collected by the smart watch 72 shows that the user 60 was recently in a patient's room in a hospital, the user 60 may be flagged as having an increased risk of transmitting a pathogen. The system 10 may accordingly increase the concentration of biocidal components in the hand cleaning fluid that is dispensed onto the user's hand 58.

Additional sensors may also be incorporated into the smartphone 70 and/or the handheld sensor device 74. For example, the smartphone 70 may incorporate a pH sensor and/or a moisture sensor that senses the pH and/or moisture level of the user's hand 58 when the user 60 is holding the smartphone 70. Alternatively, these sensors could be incorporated into a separate handheld sensor device 74, which may for example communicate with the smartphone 70 wirelessly or via a wired connection.

In some embodiments of the invention, the controller 26 may rely on data transmitted from the smartphone 70, the smart watch 72, and/or a user identification device such as an identification tag or card in order to identify the user 60. For example, the smartphone 70, the smart watch 72, and/or the user identification device may transmit a unique identification number to the controller 26, which allows the controller 26 to identify the user 60 and adjust the output fluid composition based on the corresponding user profile.

**In** some embodiments of the invention, the camera 68 may form part of a network of cameras 68 that are positioned throughout the facility, and which monitor the activities of the user 60 over time. For example, data collected by the cameras 68 may be transmitted to an interpretation system, which interprets the data in order to identify activities performed by the user 60. The interpretation system may, for example, comprise video interpretation software running on the controller 26 and/or the external server 76.

The video interpretation system may, for example, be able to identify one or more of the following activities performed by the user 60: hand cleaning; sneezing; coughing; touching a frequently touched object such as a door; entering or exiting a patient's room; touching a patient; and entering or exiting a bathroom. The controller 26 preferably uses this information in order to determine the composition of the output fluid. For example, if the system 10 determines that the user 60 has engaged in one or more activities that would increase the risk of transmitting an infection since the last time the user 60 cleaned their hands 58, the controller 26 may increase the concentration of biocidal components in the output fluid. Alternatively, if the system 10 determines that the user 60 has not engaged in any activities that would increase the risk of transmitting an infection since the last time the user 60 cleaned their hands 58, the controller 26 may decrease the concentration of biocidal components in the output fluid.

**In** some embodiments of the invention, the system 10 is preferably able to detect microbes on the user's hand 58, and adjust the composition of the output fluid based on the detected microbes. For example, the hand cleaning fluid dispenser 12 may incorporate an overflow collection and analysis tray 78, as shown in Figure 3. The tray 78 is positioned below the fluid outlet 56, so that excess fluid dispensed onto the user's hand 60 will fall into the tray 78 for analysis. The tray 78 may, for example, have the structure as described in United States Patent Publication No. US 2022/0091011 to Steltenkamp et al., published March 24, 2022, which is incorporated herein by reference. Optionally, the fluid dispenser 12 may be configured to dispense a first fluid, e.g. a sample collection fluid, onto the user's hand 58, which flows off of the user's hand 58 and into the tray 78 for analysis, and then dispense a second fluid, e.g. the output fluid, with a composition that has been adjusted based on the analysis results.

Other structures for detecting microbes and/or chemicals, such as those described in United States Patent No. 9,437,103 to Ophardt, issued September 6, 2016, and/or United States Patent Publication No. US 20210123851 to Steltenkamp et al., published April 29, 2021, could also be used. US 9,437,103 and US 20210123851 are also incorporated herein by reference.

The controller 26 is preferably configured to adjust the composition of the output fluid based on the microbes and/or chemicals detected on the user's hand 58. For example, if the analysis shows that the hand 60 is carrying a particular pathogen of concern, the output fluid may be adjusted to have a composition that targets the pathogen of concern. The system 10 may also be configured to determine the microbiome of the user's hand 58, and to adjust the composition of the output fluid accordingly. For example, the composition of the output fluid may be selected to minimize harm to the native microflora of the hand 58, while targeting non-native or allochthonous microflora.

The system 10 may also be configured to adjust the composition of the output fluid based on chemicals that are detected on the hand 58. For example, various chemical compounds, such as fatty acids, may provide an indication of the presence or absence of different types of microbes on the hand 58. The controller 26 can optionally be configured to adjust the composition of the output fluid based on the detected chemicals, in order to target pathogens of concern and minimize the harm to native microflora on the hand.

The inventors have performed a number of experiments to assess the effectiveness of different fluid compositions in killing various microorganisms. The results of these experiments are set out in the tables below.

Table 1 shows the results of an experiment testing the efficacy of various foam compositions in killing *Escherichia coli* bacteria *in vitro.* Foam 1 contained 1% sodium dodecyl sulfate, ozonized 8 times (pH 3.5); Foam 2 contained 1% sodium dodecyl sulfate, with acetic acid added until pH 3.5 is reached; Foam 3 contained 1% sodium dodecyl sulfate, with citric acid added until pH 3.5 is reached; Foam 4 contained 1% sodium dodecyl sulfate, 0.5% citric acid, pH adjusted to 3.5 with NaOH; Foam 5 contained 1% sodium dodecyl sulfate, 0.5% citric acid, pH adjusted to 4 with NaOH; and Foam 6 contained 1% sodium dodecyl sulfate, 0.5% citric acid, pH adjusted to 4.5 with NaOH.

**Table 1**

| Foam | Reduction Factor (LOG) without O₃ | Reduction Factor (LOG) with O₃ |
|---|---|---|
| 1 | 3.29 | 5.42 |
| 2 | 7.12 | 7.12 |
| 3 | 6.29 | 5.17 |
| 4 | >7.25 | >7.25 |
| 5 | >7.25 | >7.25 |
| 6 | 4.58 | 6.95 |

These experimental results suggest that the composition of a hand cleaning fluid can be tuned to target *E. coli* based at least on the following:
1) The killing rate for *E. coli* is dependent on pH and small chain acids.
2) SDS plus citric acid is more effective than SDS ozonized 8 times.
3) Ozone does not bring more effectiveness at low pH, but does at pH 4.5.
4) For non-ozonized foam: the more acidic, the better.
5) The quantity of citric acid is more important than the pH.

Table 2 shows the results of a related experiment testing the efficacy of various foam compositions in killing *Pseudomonas aeruginosa* bacteria *in vitro.* The compositions of Foams 1 to 6 are the same as in Table 1.

**Table 2**

| Foam | Reduction Factor (LOG) without O₃ | Reduction Factor (LOG) with O₃ |
|---|---|---|
| 1 | 2.33 | 2.05 |
| 2 | 6.94 | 6.47 |
| 3 | 4.30 | 4.89 |
| 4 | 7.56 | 5.62 |
| 5 | 7.06 | 5.85 |
| 6 | 5.22 | 6.08 |

These experimental results suggest that the composition of a hand cleaning fluid can be tuned to target *P. aeruginosa* based at least on the following:
1) The killing rate for *P. aeruginosa* is dependent on pH and small chain acids.
2) SDS plus citric acid is more effective than SDS ozonized 8 times.
3) Ozone does not bring more effectiveness for *P. aeruginosa.*
4) pH has no influence on the effectiveness of ozonized foam.
5) For non-ozonized foam: the more acidic, the better.
6) The quantity of citric acid is more important than the pH.

Table 3 shows the results of a related experiment testing the efficacy of various foam compositions in killing *Bacillus subtilis* spores *in vitro.* The compositions of Foams 1 to 6 are the same as in Table 1. Foam 7 contained 70% isopropanol.

**Table 3**

| Foam | Reduction Factor (LOG) without O₃ | Reduction Factor (LOG) with O₃ |
|---|---|---|
| 1 | >7 | >7 |
| 2 | >7 | >7 |
| 3 | >7 | >7 |
| 4 | >7 | >7 |
| 5 | >7 | >7 |
| 6 | >7 | >7 |
| 7 | 0 | 0 |

These experimental results suggest that the composition of a hand cleaning fluid can be tuned to target *Bacillus subtilis* spores based at least on the following:
1) Isopropanol is not effective at killing *Bacillus subtilis* spores.
2) SDS solutions are effective.

The experimental results reproduced in Tables 1 to 3 suggest the following general conclusions:
1) The SDS solutions tested are 100% effective against *Bacillus subtilis* spores.
2) Of the solutions tested, 0.5% citric acid is the most effective against bacteria.
3) Effectiveness decreases at pH 4.5, but is partially restored by ozone.

Additional experiments were also performed to test the effect of adding glycerin to the compositions, which are summarized in the tables below.

Table 4 shows the effect of 1% glycerin on the killing rates for *E. coli* and *P*. *aeruginosa in vitro.* Foam 8 contained 0.5% sodium dodecyl sulfate and 0.5% citric acid; Foam 9 contained 0.5% sodium dodecyl sulfate, 0.5% citric acid, and ozone; Foam 10 contained 0.5% sodium dodecyl sulfate, 0.5% citric acid, and 1% glycerin; and Foam 11 contained 0.5% sodium dodecyl sulfate, 0.5% citric acid, 1% glycerin, and ozone. Foams 8 to 11 each had a pH of 4.5.

**Table 4**

| Foam | Reduction Factor (LOG) for *E. coli* | Reduction Factor (LOG) for *P. aeruginosa* |
|---|---|---|
| 8 | 1.1 | 5.7 |
| 9 | 4.9 | 6.2 |
| 10 | 4.9 | 7.2 |
| 11 | 7.2 | 7.7 |

These experimental results suggest that adding glycerin improves the antibacterial effect, and adding ozone enhances the killing of *E. coli.*

Additional experimental results are reproduced in Tables 5 and 6. Table 5 shows the reduction factor for various foams for *E*. *coli,* and Table 6 shows the reduction factor for various foams for *P. aeruginosa.* Foam 12 contained 0.5% sodium dodecyl sulfate and 0.5% citric acid; Foam 13 contained 0.5% sodium dodecyl sulfate, 0.5% citric acid, and 1% glycerin; and Foam 14 contained 0.5% sodium dodecyl sulfate, 0.5% citric acid, and 10% glycerin. Foams 12 to 14 each has a pH of 4.5. The efficacy of the foams was tested *in vitro.*

**Table 5**

| Foam | Reduction Factor (LOG) without O₃ | Reduction Factor (LOG) with O₃ |
|---|---|---|
| 12 | 2.2 | 5.4 |
| 13 | 3.5 | 6.7 |
| 14 | 3.0 | 7.6 |

**Table 6**

| Foam | Reduction Factor (LOG) without O₃ | Reduction Factor (LOG) with O₃ |
|---|---|---|
| 12 | 5.5 | 6.5 |
| 13 | 5.8 | 6.6 |
| 14 | 3.9 | 5.7 |

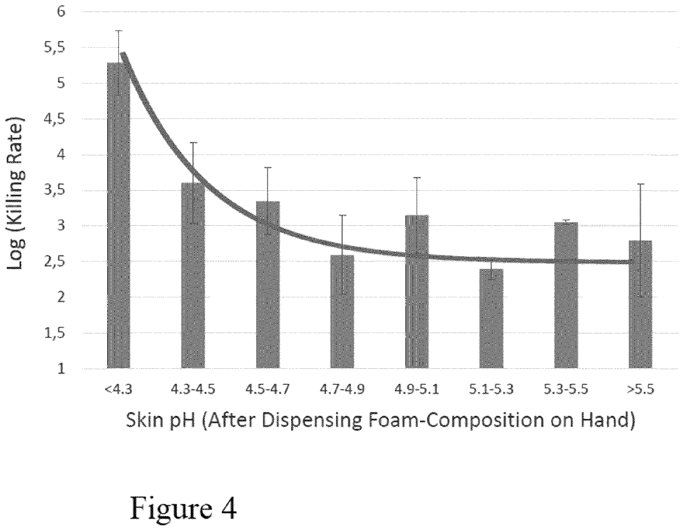

Additional experiments were performed to assess the effect of skin pH on the killing rate of hand cleaning fluids. The pH of the hands was assessed after dispensing a foam composition onto the hands. The results are summarized in the graph shown in Figure 4.

As can be seen in Figure 4, a trend of exponentially decreasing effectiveness can be seen as hand pH increases. This trend was found to be independent of test person, test solution, and other conditions. The results suggest that targeting a pH of less than 4.7, and preferably around 4.3, of the hand after the output fluid has been dispensed onto the hand can improve the effectiveness of the hand cleaning solution. Preferably, the pH of the hand remains above 4, and more preferably above 4.2, to reduce the risk of skin irritation or damage.

In some preferred embodiments of the invention, the controller 26 adjusts the composition of the output fluid so as to achieve a target pH on the hand 58 after the fluid has been dispensed onto the hand 58. For example, the controller 26 may receive information about the pH of the hand 58 from a pH meter before the fluid has been dispensed onto the hand 58, and then adjust the pH of the output fluid based on the measured pH of the hand 58, in order to achieve the target pH after the fluid has been dispensed.

Additional tests performed by the inventors found that the killing rate of an SDS solution was improved by ozonizing the fluid multiple times. It is believed that the enhanced killing rate from ozonizing the fluid multiple times may be explained at least partially by reactions between the ozone and the SDS generating small carboxylic acids that lower the pH of the solution. Subsequent experiments found that the killing rate is improved by using citric acid or acetic acid to lower the pH, instead of ozonizing the fluid multiple times (see Tables 1 and 2 above).

It will be understood that, although various features of the invention have been described with respect to one or another of the embodiments of the invention, the various features and embodiments of the invention may be combined or used in conjunction with other features and embodiments of the invention as described and illustrated herein.

The invention is not limited to the particular structures of the preferred embodiments that have been shown in the drawings. Rather, any functionally equivalent structures could be used.

Although the examples have described the fluid dispenser 12 as generating the output fluid from four input substances, the number of input substances could be increased or decreased as desired. Any suitable input substances could be used, including input substances that are in liquid, gaseous, or solid form. Optionally, the dispenser 12 could have a first input reservoir 32 containing a concentrated hand cleaning solution, which is then diluted with water to generate the output fluid.

Although the output fluid has been described in the preferred embodiments as a foam, it could alternatively have any suitable form including a liquid, a gel, an emulsion, or a combination thereof.

Preferably, the fluid dispenser 12 includes a lock out mechanism that prevents output fluid from being dispensed if any of the input reservoirs 32, 38, 44 are empty. The lock out mechanism may be mechanical and/or electronic. For example, an electronic mechanism may include sensors that sense the fluid levels in each of the input reservoirs 32, 38, 44, and communicate this information to the controller 26. The controller 26 may be configured to automatically request service whenever one of the input reservoirs 32, 38, 44 is running low or empty and needs to be refilled or replaced. If any input reservoir 32, 38, 44 becomes empty, the controller 26 preferably prevents the fluid dispenser 12 from dispensing any further output fluid until the input reservoir 32, 38, 44 has been refilled or replaced.

An example of a mechanical lockout mechanism is described in United States Patent No. 10,267,305 to Ophardt et al., issued April 23, 2019, which is incorporated herein by reference. A similar mechanical lock out mechanism could be incorporated into the fluid dispenser 12 in order to prevent the fluid dispenser 12 from dispensing fluid when any of the input reservoirs 32, 38, 44 is empty.

The fluid dispenser 12 is preferably capable of operating independently, without requiring service to refill or replace the input reservoirs 32, 38, 44 for extended periods of time. For example, if the dispenser 12 is configured to draw water from the tap system, a 10L reservoir of 130g/L SDS could last for about 5 years before needing to be refilled or replaced.

In one example of the invention, the fluid dispenser 12 could include a first input reservoir 32 containing 850 mL or 1 L of SDS solution (13.39% or 133.9 g/L or 0.46 mol/L); a second input reservoir 38 containing 25L of water; a third input reservoir 44 containing 850 mL or 1 L of citric acid solution (13.39% or 133.9 g/L or 0.69 mol/L); and an ozone generator 22 configured to provide from 20 ppm to 2000pm of ozone. Optionally, the dispenser 12 may include a mixing chamber 52 for mixing the SDS solution, the water, and the citric acid solution. The mixing chamber 52 may, for example, hold approximately 15 mL of the pre-mixed fluid, or enough for approximately 10 dispensing events. The mixing chamber 52 may employ active or passive mixing, such as passive vortices mixing or spiral-mixing or active magnetic stirring. Optionally, the mixing chamber 52 may be configured to discard the remaining pre-mixed fluid after the user 60 has finished using the dispenser 12, and before a subsequent user 60 uses the dispenser 12. The system 10 may, for example, use proximity sensors to detect when a user 60 approaches and departs from the area surrounding the dispenser 12. The system 10 may be configured to prepare the pre-mixed fluid as soon as the user 60 approaches the dispenser 12, and/or to discard any fluid remaining in the mixing chamber 52 when the user 60 moves away from the dispenser 12. In one preferred example, the fluid dispenser 12 may be configured to deliver 0.5 mL of the SDS solution, 14 mL of water, and 0.5 mL of the citric acid solution to the mixing chamber 52, to generate a premixed fluid having 0.5% w/w SDS and 0.5% w/w citric acid. The premixed fluid can then be combined with gas from the ozone generator 22 to generate foam that is dispensed onto the user's hand 58. In one preferred example, the ozone generator 22 generates approximately 600 ppm or 500 ppm of ozone. Optionally, additional input reservoirs containing additional input components, such as glycerin, could also be added to the dispenser 12. Optionally, the mixing chamber 52 (which may also be referred to as a pre-reservoir) is configured to heat or cool the pre-mixed fluid.

In another example, the fluid dispenser 12 could include a first input reservoir 32 containing 850 mL or 1 L of a solution containing both SDS and citric acid (e.g. 13.39% SDS and 13.39% citric acid); and a second input reservoir 38 containing water.

The system 10 preferably includes multiple fluid dispensers 12 that are arranged throughout a facility, such as a hospital. The dispensers 12 are preferably able to communicate with each other directly or via a communication hub or external server 76. In some embodiments of the invention, the controller 26 in one dispenser 12 may adjust the composition of the output fluid based on data received from one or more of the other dispensers 12 in the network. For example, if one dispenser 12 detects a pathogen on a user's hand 58, the next dispenser 12 that the user 60 uses may be configured to adjust the composition of the output fluid to target the detected pathogen.

All concentrations written herein as a percentage are by weight (w/w), unless otherwise indicated.

In the *in vitro* experiments summarized above, the following method was used: the bacterial culture was serially diluted (1 LOG); the tested foam was added to 50 µL of the diluted solution and stirred for 15 seconds; the resulting mixture was transferred to an agar plate and incubated for 24 hours before counting the bacterial colonies (CFUs). The LOG reduction factor was calculated as the LOG bacteria before treatment minus the LOG bacteria after treatment.

The experimental results shown in Figure 4 were collected using the following method, based on the DIN EN-1500 testing protocol: hands were washed with a soft soap, and then immersed in the contamination liquid for 5 seconds; the hands were then dried in air for 3 minutes, before fingers were rubbed in sterile culture medium to collect the pre-treatment CFU counts. The hands were then dried before being rubbed with the test foam for 30 seconds; finger tips were then placed in a neutralizer before being rubbed in sterile culture medium to collect the post-treatment CFU counts. The LOG reduction factor was calculated as the LOG pre-treatment count minus the LOG post-treatment count.

Although the examples shown in the drawings include a single component (ozone generator 22) for adding gas to the output fluid, additional components for adding gas could be incorporated into the dispenser 12. For example, the dispenser 12 could include separate components for selectively delivering different gases such as ozone, oxygen, nitrogen, air, and/or argon. Any suitable gas or gases could be used in generating the output foam. Alternatively, the output fluid could be delivered as a liquid, without any gas added thereto.

In one preferred embodiment, the hand cleaning fluid includes sodium dodecyl sulfate (SDS), citric acid, glycerin, and water. In another preferred embodiment, the fluid is mixed with a gas to generate foam, and the gas preferably includes ozone. In one preferred embodiment, the fluid includes 0.5% SDS, 0.5% citric acid, and 1% glycerin. In another preferred embodiment, the fluid includes 1% SDS, 1% citric acid, and 1% glycerin.

In some embodiments of the invention, the fluid adjustor includes components that are external to the fluid dispenser 12, such as an external server 76 that determines or assists in determining the composition of the output fluid. In other embodiments of the invention, all components of the fluid adjustor may be incorporated into the fluid dispenser 12 itself. Any suitable mechanisms for adjusting the composition of the output fluid could be used. For example, the input dispensers 16, 18, 20 may each include an electric motor for activating their pump mechanisms 34, 40, 46, and the quantity of input fluid delivered by each input dispenser 16, 18, 20 may be adjusted by increasing or decreasing the activation time of their respective electric motors. The term "fluid adjustor" as used herein is intended to refer to all components of the system 10 that are involved in adjusting the composition of the output fluid. Preferably, the fluid adjustor is configured to automatically adjust the composition of the output fluid based on one or more factors, such as a health status of the user and/or an identity of the user. The term "automatically" as used herein means that the composition is adjusted by the system 10 without requiring any user intervention to set or select the composition at the time the output fluid is being dispensed.

In some embodiments of the invention, the composition of the output fluid may be adjusted based, at least in part, on the time of day and/or the date that the fluid is being dispensed. For example, the system 10 may be configured to dispense a fluid that has increased antimicrobial qualities during times of the year when there is increased transmission of infectious diseases, e.g. during flu season. The system 10 may also be configured to dispense fluids that have increased moisturizing properties at times of day when users may be at increased risk of having dry hands, e.g. near the end of a shift for nurses at a hospital or healthcare facility. Preferably the system 10 includes a time keeping device or clock that monitors the time of day and the date that the output fluid is dispensed.

In some embodiments of the invention, the system 10 may be configured to adjust the composition based, at least in part, on the schedule of the user. The schedule may, for example, be stored on the external server 76 and/or may be communicated to the system 10 from a smart watch 72 or smart phone 70 carried by the user. The schedule may, for example, include times when the user will be physically interacting with patients in a healthcare facility, and other times when the user will be doing paper work or other work that does not involve interacting with patients. The system 10 may be configured to dispense a fluid having an increased antimicrobial activity when the user is scheduled to be interacting with patients, and a fluid having a decreased antimicrobial activity when the user is scheduled to not be interacting with patients.

Any sensor described herein that may be used by the system 10 to determine or predict the health status of the user may be referred to as a health status sensor. The health status sensor may comprise, for example, an optical detection system, such as a camera. The system 10 may include a health status determination/prediction unit that determines or predicts the health status of the user based, for example, on data from one or more health status sensors. The health status determination unit may be incorporated into the controller 26 and/or the external server 76. The controller 26 and/or external server 76 may also function as a biometric identification device that determines the identity of the user based on biometric data. The biometric data may include, for example, photographs or videos of the user taken by a camera 68; fingerprint data; iris scan data, and/or voice recognition data. The controller 26 and/or external server 76 may also function as a user history determination unit that is configured to determine the history of the user. The user history may be determined based on one or more of: location data for the user, dispenser use data for the user, activity data for the user, and human resource data for the user. The human resource data may include, for example, records of patients the user has physically interacted with, and records of the shifts the user has worked. The controller 26 and/or the external server 76 may also form part of a video recording and interpretation system, which may also include cameras 68 positioned throughout the facility. The video recording and interpretation system is preferably configured to collect video data of the user and to identify activities performed by the user based, at least in part, on the video data. The controller 26 and/or the external server 76 may also store a user schedule database, a user allergy database, and/or a chemical sensitivity database. The controller 26 and/or the external server 76 may also function as an infection history determination unit that determines the history of infections in the vicinity of the hand cleaning fluid dispenser 12 based on infection data. The infection data may include data collected by a network of infection detection devices and/or public health data received from a public health authority. The network of infection detection devices could include a network of fluid dispensers 12 that are capable of detecting infections and/or microbes.

In some embodiments of the invention, the composition of the output fluid may be selected based on input from the user. For example, the user may be able to select their preferred fluid composition on their smartphone 70 or smart watch 72, which transmits their selection to the controller 26. In some embodiments of the invention, a facility administrator may be able to select the composition of the output fluid, for example by inputting instructions to the external server 76.

In some embodiments of the invention, the composition of the output fluid may be adjusted to be more aggressive at killing pathogens, for example during flu season, an outbreak, and/or an epidemic. The composition of the output fluid may also be adjusted to add additional hand care components, such as moisturizer, depending on the user's needs and/or preferences. In some embodiments of the invention, one or more of the input fluids may be a concentrated solvent, such as alcohol or chlorine or any other suitable disinfection solution. In some embodiments of the invention, the composition of the output fluid may be adjusted based on the risk of getting/having/transmitting an infection. In some embodiments of the invention, the composition of the output fluid may be adjusted based on the specific pathogens that are known or expected to be circulating in the location where the dispenser is located. In some embodiments of the invention, the composition of the output fluid may be adjusted based on the location(s) where the user lives, works, and/or travels to. In some embodiments of the invention, the composition of the output fluid may be adjusted based on the medical history and/or medical records of the user and/or the medical history and/or medical records of another individual or group or population, including for example individuals that the user has interacted with in the past or is expected to interact with in the future. In some embodiments of the invention, carbon dioxide may be used as one of the input substances. The carbon dioxide may be used, for example, to help stabilize ozone. In some embodiments of the invention, the composition of the output fluid may be adjusted to protect resident bacteria of the skin/hand. In some embodiments of the invention, the output fluid comprises at least 50 ppm, at least 200 ppm, or at least 500 ppm of ozone. In some embodiments of the invention, the dispenser comprises a gas reservoir. The gas reservoir may contain a supply of any gas that may be used as an input component, such as air, oxygen, ozone, carbon dioxide, and/or argon. In some embodiments of the invention, the composition of the output fluid may be adjusted based on the kind of infection(s) and/or pathogen(s) that have been detected or are predicted to be present in the area where the dispenser is located and/or have been detected or are predicted to be present in or on the user. In some embodiments of the invention, the composition of the output fluid may be adjusted based on the presence of an epidemic, the characteristics of the epidemic, the presence of a pandemic, and/or the characteristics of the pandemic. In some embodiments of the invention, the composition of the output fluid may be adjusted based on whether or not it is flu (influenza) season in the area where the hand cleaning fluid dispenser is located. In some embodiments of the invention, the composition of the output fluid may be adjusted based on the posture, the movement, and/or the gestures of the user. In some embodiments of the invention, the composition of the output fluid may be adjusted based on data collected by at least one contamination sensor that detects contamination and/or pathogens that are on the user's hand and/or have recently been on the user's hand.

Although this disclosure has described and illustrated certain preferred embodiments of the invention, it is to be understood that the invention is not restricted to these particular embodiments. Rather, the invention includes all embodiments which are functional or mechanical equivalents of the specific embodiments and features that have been described and illustrated herein.

## Claims

1. A hand cleaning system (10) comprising:
a hand cleaning fluid dispenser (12) configured to dispense an output fluid onto a hand (58) of a user (60); and
a fluid adjustor configured to automatically adjust a composition of the output fluid based, at least in part, on:
a health status of the user (60);
a predicted health status of the user (60);
a pH of the hand (58);
an identity of the user (60);
a history of the user (60);
a schedule of the user (60);
a history of infections detected in a vicinity of the hand cleaning fluid dispenser (12);
an infection profile for an area where the hand cleaning fluid dispenser (12) is located;
a predicted likelihood that the user (60) has an infection;
a microbiome of the hand (58);
a time of day that the output fluid is dispensed;
a date that the output fluid is dispensed;
microbes detected on the hand (58);
a moisture level of the hand (58);
a concentration of one or more chemicals on the hand (58);
an allergy profile of the user (60); or
a chemical sensitivity profile of the user (60).

2. The hand cleaning system (10) according to claim 1, wherein the hand cleaning system (10) further comprises at least one health status sensor.

3. The hand cleaning system (10) according to claim 2, wherein the at least one health status sensor comprises at least one of:
a body temperature sensor;
a wearable sensor that is incorporated into a wearable device; and
a camera (68).

4. The hand cleaning system (10) according to claim 3, wherein the hand cleaning system (10) further comprises a health status determination unit that is configured to determine the health status of the user (60) based, at least in part, on data collected by the camera (68); and
wherein the health status determination unit is configured to interpret the data collected by the camera (68) to recognize at least one of: coughing, sneezing, nose blowing, vomiting, bleeding, and frequency of bathroom use.

5. The hand cleaning system (10) according to any one of claim 1 to 4, wherein the hand cleaning system (10) further comprises a pH determination device that is configured to determine the pH of the hand (58).

6. The hand cleaning system (10) according to claim 5, wherein the pH determination device comprises at least one of:
a pH meter;
a pH meter that is incorporated into the hand cleaning fluid dispenser (12);
a pH meter that is carried by the user (60); and
a pH sensor that is incorporated into a smart phone (70).

7. The hand cleaning system (10) according to any one of claims 1 to 6, wherein the hand cleaning system (10) further comprises a biometric identification device that is configured to determine the identity of the user (60) based on biometric data.

8. The hand cleaning system (10) according to any one of claims 1 to 7, wherein the hand cleaning system (10) further comprises an electronic identification device that is configured to determine the identity of the user (60) based on identification data received from a user identification device carried by the user (60); and
wherein the user identification device comprises at least one of: a smart watch (72), a smart phone (70), an identification tag, and an identification card.

9. The hand cleaning system (10) according to any one of claims 1 to 8, wherein the hand cleaning system (10) further comprises a user history determination unit that is configured to determine the history of the user (60) based, at least in part, on at least one of: location data for the user (60), dispenser use data for the user (60), activity data for the user (60), and human resource data for the user (60).

10. The hand cleaning system (10) according to claim 9, wherein the hand cleaning system (10) further comprises a video recording and interpretation system that is configured to collect video data of the user (60) and to identify activities performed by the user (60) based, at least in part, on the video data.

11. The hand cleaning system (10) according to any one of claims 1 to 10, wherein the fluid adjustor is incorporated into the hand cleaning fluid dispenser (12).

12. The hand cleaning system (10) according to any one of claims 1 to 11, wherein the hand cleaning system (10) further comprises a microbe detection device that is configured to detect microbes on the hand (58).

13. The hand cleaning system (10) according to any one of claims 1 to 12, wherein the hand cleaning system (10) further comprises a moisture detecting device that is configured to detect the moisture level of the hand (58);
wherein the moisture detecting device comprises a contactless moisture meter; and
wherein the moisture detecting device is incorporated into the hand cleaning fluid dispenser (12).

14. The hand cleaning system (10) according to any one of claims 1 to 13, wherein the hand cleaning system (10) further comprises a chemical detecting device that is configured to detect one or more chemicals on the hand (58); and
wherein the chemical detecting device is configured to determine the concentration of fatty acids on the hand (58).

15. The hand cleaning system (10) according to any one of claims 1 to 14, wherein the fluid adjustor adjusts the composition of the output fluid by selecting a quantity of one or more input substances that are combined to form the output fluid;
wherein the input substances comprise at least one of:
water;
a soap;
an alcohol;
a solvent;
a disinfectant solution;
chlorine;
a surfactant;
an acid;
a base;
a gas;
sodium dodecyl sulfate;
citric acid;
acetic acid;
sodium hydroxide;
ozone;
air;
nitrogen;
argon;
carbon dioxide;
a moisturizer; and
glycerin;
wherein the hand cleaning system (10) further comprises at least one mixing chamber (52) for combining the input substances.
